# EUROPEAN PATENT APPLICATION

(11) **EP 3 608 426 A1**
(43) Date of publication of application: **12.02.2020**
(21) Application number: 18188489.1
(22) Date of filing: 10.08.2018
(51) Int. Cl.: C12Q 1/6897, C12N 15/09

(54) **RECOMBINANT NUCLEIC ACID CONSTRUCT**

(71) Applicant: Pantherna Therapeutics GmbH, 16761 Hennigsdorf (DE)
(72) Inventor: The designation of the inventor has not yet been filed
(74) Representative: Bohmann, Armin K.

(57) **Abstract**

The present invention is related to a recombinant nucleic acid construct comprising in 5'-> 3' direction
- a 5' UTR,
- a coding region coding for an effector molecule, and
- a 3' UTR,
wherein the 5' UTR is selected from the group comprising a 5' UTR of a gene coding for MCP-1 or a derivative thereof having a nucleotide identity of at least 85 %, a 5' UTR of a gene coding for Ang-2 or a derivative thereof having a nucleotide identity of at least 85 %, a 5' UTR of a gene coding for RPL12s.c. or a derivative thereof having a nucleotide identity of at least 85 %, a 5' UTR of a gene coding for HSP70 or a derivative thereof having a nucleotide identity of at least 85 %, a 5' UTR of a gene coding for H3.3. or a derivative thereof having a nucleotide identity of at least 85 %, a 5' UTR of a gene coding for Galectin-9 or a derivative thereof having a nucleotide identity of at least 85 %, a 5' UTR of a gene coding for GADD34 or a derivative thereof having a nucleotide identity of at least 85 %, a 5' UTR of a gene coding for EDN1 or a derivative thereof having a nucleotide identity of at least 85 %, a 5' UTR of a gene coding for HSP70m5 or a derivative thereof having a nucleotide identity of at least 85 %, a 5' UTR of a gene coding for E-selectin or a derivative thereof having a nucleotide identity of at least 85 % a 5' UTR of a gene coding for ICAM-1 or a derivative thereof having a nucleotide identity of at least 85 %, a 5' UTR of a gene coding for IL-6 or a derivative thereof having a nucleotide identity of at least 85 % and a 5' UTR of a gene coding for vWF or a derivative thereof having a nucleotide identity of at least 85 %;
wherein 3' UTR is selected from the group comprising a 3' UTR of a gene coding for MCP-1 or a derivative thereof having a nucleotide identity of at least 85 %, a 3' UTR of a gene coding for vWF or a derivative thereof having a nucleotide identity of at least 85 %, a 3' UTR of a gene coding for RPL12s.c. or a derivative thereof having a nucleotide identity of at least 85 %, a 3' UTR of a gene coding for HSP70 or a derivative thereof having a nucleotide identity of at least 85 %, a 3' UTR of a gene coding for H3.3. or a derivative thereof having a nucleotide identity of at least 85 %, a 3' UTR of a gene coding for GADD34 or a derivative thereof having a nucleotide identity of at least 85 %, a 3' UTR of a gene coding for EDN1 or a derivative thereof having a nucleotide identity of at least 85 %, and a 3' UTR of a gene coding for IL-6 or a derivative thereof having a nucleotide identity of at least 85 %,
wherein the effector molecule is effective in restoring a cellular function of a cell or is effective in exercising a therapeutic effect in or on a cell, and
wherein the recombinant nucleic acid construct is different from a wild type mRNA coding for the effector molecule.

## Description

The present invention is related to a recombinant nucleic acid construct, a vector, preferably an expression vector, comprising a recombinant nucleic acid construct, a cell comprising the recombinant nucleic acid construct and/or the vector, a delivery vehicle comprising the recombinant nucleic acid construct, a pharmaceutical composition comprising the recombinant nucleic acid construct, the recombinant nucleic acid construct for use in a method for the treatment and/or prevention of a disease and the recombinant nucleic acid construct for use in a method for restoring a cellular function of a cell.

Nucleic acid molecules serve various purposes in biological systems. One out of several purposes is to store genetic information, another one is to convey genetic information from deoxyribonucleic acid to the ribosome where such genetic information is translated into an amino acid sequence. The latter step involves the use of messenger ribonucleic acid (mRNA).

mRNA shows a very basic design in eukaryotic cells and typically comprises, in 5'-> 3' direction, a Cap structure, a 5' untranslated region (5' UTR), a coding sequence typically starting with a AUG codon attached to a coding sequence (CDS) terminating with a stop codon, a 3' untranslated region (3' UTR) and a poly-A-tail.

mRNA based therapy have been proposed as therapeutics since the beginning of the biotech. In principle, the administered mRNA sequence can cause a cell to make a protein, which in turn could directly treat a disease or could function as a vaccine; more indirectly the protein could interfere with a elements of pathway in such way that the pathway is either inhibited or stimulated, thereby treating or ameliorating a disease.

The problem underlying the present invention is the provision of a recombinant nucleic acid construct, preferably of an mRNA, whereby such recombinant nucleic acid construct allows for high expression of the coding sequence of the recombinant nucleic acid construct.

Another problem underlying the present invention is the provision of a recombinant nucleic acid construct, preferably of an mRNA, whereby such recombinant nucleic acid construct allows for the expression of the coding sequence of the recombinant nucleic acid construct in an endothelial cell.

These and other problems underlying the present invention are solved by the subject matter of the attached independent claims; preferred embodiments may be taken from the attached dependent claims.

More specifically, the problem underlying the present invention is solved in a first aspect, which is also the first embodiment of the first aspect, by a recombinant nucleic acid construct comprising in 5'-> 3' direction
- a 5' UTR,
- a coding region coding for an effector molecule, and
- a 3' UTR,
wherein the 5' UTR is selected from the group comprising a 5' UTR of a gene coding for MCP-1 or a derivative thereof having a nucleotide identity of at least 85 %, a 5' UTR of a gene coding for Ang-2 or a derivative thereof having a nucleotide identity of at least 85 %, a 5' UTR of a gene coding for RPL12s.c. or a derivative thereof having a nucleotide identity of at least 85 %, a 5' UTR of a gene coding for HSP70 or a derivative thereof having a nucleotide identity of at least 85 %, a 5' UTR of a gene coding for H3.3. or a derivative thereof having a nucleotide identity of at least 85 %, a 5' UTR of a gene coding for Galectin-9 or a derivative thereof having a nucleotide identity of at least 85 %, a 5' UTR of a gene coding for GADD34 or a derivative thereof having a nucleotide identity of at least 85 %, a 5' UTR of a gene coding for EDN1 or a derivative thereof having a nucleotide identity of at least 85 %, a 5' UTR of a gene coding for HSP70m5 or a derivative thereof having a nucleotide identity of at least 85 %, a 5' UTR of a gene coding for E-selectin or a derivative thereof having a nucleotide identity of at least 85 % a 5' UTR of a gene coding for ICAM-1 or a derivative thereof having a nucleotide identity of at least 85 %, a 5' UTR of a gene coding for IL-6 or a derivative thereof having a nucleotide identity of at least 85 % and a 5' UTR of a gene coding for vWF or a derivative thereof having a nucleotide identity of at least 85 %;
wherein 3' UTR is selected from the group comprising a 3' UTR of a gene coding for MCP-1 or a derivative thereof having a nucleotide identity of at least 85 %, a 3' UTR of a gene coding for vWF or a derivative thereof having a nucleotide identity of at least 85 %, a 3' UTR of a gene coding for RPL12s.c. or a derivative thereof having a nucleotide identity of at least 85 %, a 3' UTR of a gene coding for HSP70 or a derivative thereof having a nucleotide identity of at least 85 %, a 3' UTR of a gene coding for H3.3. or a derivative thereof having a nucleotide identity of at least 85 %, a 3' UTR of a gene coding for GADD34 or a derivative thereof having a nucleotide identity of at least 85 %, a 3' UTR of a gene coding for EDN1 or a derivative thereof having a nucleotide identity of at least 85 %, and a 3' UTR of a gene coding for IL-6 or a derivative thereof having a nucleotide identity of at least 85 %,
wherein the effector molecule is effective in restoring a cellular function of a cell or is effective in exercising a therapeutic effect in or on a cell, and
wherein the recombinant nucleic acid construct is different from a wild type mRNA coding for the effector molecule.

In a second embodiment of the first aspect, which is also an embodiment of the first embodiment of the first aspect,
a) the 5' UTR is a 5' UTR of a gene coding for HSP70 or a derivative thereof having a nucleotide identity of at least 85 %, and wherein the 3' UTR is selected from the group comprising a 3' UTR of a gene coding for HSP70 or a derivative thereof having a nucleotide identity of at least 85 %, a 3' UTR of a gene coding for RPL12s.c. or a derivative thereof having a nucleotide identity of at least 85 %, a 3' UTR of a gene coding for vWF or a derivative thereof having a nucleotide identity of at least 85 % and a 3' UTR of a gene coding for MCP-1 or a derivative thereof having a nucleotide identity of at least 85 %,
b) the 5' UTR is a 5' UTR of a gene coding for RPL12s.c. or a derivative thereof having a nucleotide identity of at least 85 %, and wherein the 3' UTR is selected from the group comprising a 3' UTR of a gene coding for HSP70 or a derivative thereof having a nucleotide identity of at least 85 %, a 3' UTR of a gene coding for RPL12s.c. or a derivative thereof having a nucleotide identity of at least 85 %, a 3' UTR of a gene coding for vWF or a derivative thereof having a nucleotide identity of at least 85 % and a 3' UTR of a gene coding for MCP-1 or a derivative thereof having a nucleotide identity of at least 85 %,
c) the 5' UTR is a 5' UTR of a gene coding for ANG-2 or a derivative thereof having a nucleotide identity of at least 85 %, and wherein the 3' UTR is selected from the group comprising a 3' UTR of a gene coding for HSP70 or a derivative thereof having a nucleotide identity of at least 85 %, a 3' UTR of a gene coding for RPL12s.c. or a derivative thereof having a nucleotide identity of at least 85 %, a 3' UTR of a gene coding for vWF or a derivative thereof having a nucleotide identity of at least 85 % and a 3' UTR of a gene coding for MCP-1 or a derivative thereof having a nucleotide identity of at least 85 %, or
d) the 5' UTR is a 5' UTR of a gene coding for MCP-1 or a derivative thereof having a nucleotide identity of at least 85 %, and wherein the 3' UTR is selected from the group comprising a 3' UTR of a gene coding for HSP70 or a derivative thereof having a nucleotide identity of at least 85 %, a 3' UTR of a gene coding for RPL12s.c. or a derivative thereof having a nucleotide identity of at least 85 %, a 3' UTR of a gene coding for vWF or a derivative thereof having a nucleotide identity of at least 85 % and a 3' UTR of a gene coding for MCP-1 or a derivative thereof having a nucleotide identity of at least 85 %.

In a third embodiment of the first aspect, which is also an embodiment of the first embodiment of the first aspect,
a) the 3'UTR is a 3' UTR of a gene coding for HSP70 or a derivative thereof having a nucleotide identity of at least 85 % and the 5' UTR is selected from the group comprising a 5' UTR of a gene coding for HSP70 or a derivative thereof having a nucleotide identity of at least 85 % a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %,
b) the 3'UTR is a 3' UTR of a gene coding for RPL12s.c. or a derivative thereof having a nucleotide identity of at least 85 % and the 5' UTR is selected from the group comprising a 5' UTR of a gene coding for HSP70 or a derivative thereof having a nucleotide identity of at least 85 % a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %,
c) the 3'UTR is a 3' UTR of a gene coding for vWF or a derivative thereof having a nucleotide identity of at least 85 % and the 5' UTR is selected from the group comprising a 5' UTR of a gene coding for HSP70 or a derivative thereof having a nucleotide identity of at least 85 % a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %, or
d) the 3'UTR is a 3' UTR of a gene coding for MCP-1 or a derivative thereof having a nucleotide identity of at least 85 % and the 5' UTR is selected from the group comprising a 5' UTR of a gene coding for HSP70 or a derivative thereof having a nucleotide identity of at least 85 %, a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %.

In a fourth embodiment of the first aspect, which is also an embodiment of the first, second and third embodiment of the first aspect, the construct is one selected from the group comprising
a construct, wherein the 5' UTR is a 5' UTR of a gene coding for MCP-1 or a derivative thereof having a nucleotide identity of at least 85 %, and the 3' UTR is a 3' UTR of a gene coding for MCP-1 or a derivative thereof having a nucleotide identity of at least 85 %,
a construct, wherein the 5' UTR is a 5' UTR of a gene coding for ANG-2 or a derivative thereof having a nucleotide identity of at least 85 %, and the 3' UTR is a 3' UTR of a gene coding for vWF or a derivative thereof having a nucleotide identity of at least 85 %,
a construct, wherein the 5' UTR is a 5' UTR of a gene coding for RPL12s.c. or a derivative thereof having a nucleotide identity of at least 85 %, and the 3' UTR is a 3' UTR of a gene coding for RPL12s.c. or a derivative thereof having a nucleotide identity of at least 85 %,
a construct, wherein the 5' UTR is a 5' UTR of a gene coding for HSP70 or a derivative thereof having a nucleotide identity of at least 85 %, and the 3' UTR is a 3' UTR of a gene coding for HSP70 or a derivative thereof having a nucleotide identity of at least 85 %,
a construct, wherein the 5' UTR is a 5' UTR of a gene coding for H3.3. or a derivative thereof having a nucleotide identity of at least 85 %, and the 3' UTR is a 3' UTR of a gene coding for H3.3. or a derivative thereof having a nucleotide identity of at least 85 %, and
a construct, wherein the 5' UTR is a 5' UTR of a gene coding for Galectin 9 or a derivative thereof having a nucleotide identity of at least 85 %, and the 3' UTR is a 3' UTR of a gene coding for Galectin 9 or a derivative thereof having a nucleotide identity of at least 85 %.

In a fifth embodiment of the first aspect, which is also an embodiment of the first, second, third and fourth embodiment of the first aspect, the construct comprises a poly-A tail, preferably at the 3' terminal end of the recombinant nucleic acid construct.

In a sixth embodiment of the first aspect, which is also an embodiment of the first, second, third, fourth and fifth embodiment of the first aspect, the construct comprises a CAP structure, preferably at the 5' terminal end of the recombinant nucleic acid construct.

In a seventh embodiment of the first aspect, which is also an embodiment of the first, second, third, fourth and fifth embodiment of the first aspect, the construct comprises a IRES (internal ribosomal entry site) sequence, preferably at the 5' terminal end of the recombinant nucleic acid construct.

In an eighth embodiment of the first aspect, which is also an embodiment of the first, second, third, fourth, fifth, sixth and seventh embodiment of the first aspect, the construct comprises nucleic acid sequence coding for a signal peptide, preferably the signal peptide is in-frame with nucleic acid sequence coding for a signal peptide and is arranged between the 5' UTR and the coding region coding for an effector molecule.

In a ninth embodiment of the first aspect, which is also an embodiment of the eighth embodiment of the first aspect, the signal peptide allows secretion of the effector molecule.

In a tenth embodiment of the first aspect, which is also an embodiment of the eighth and the ninth embodiment of the first aspect, the nucleotide sequence coding for a signal peptide is selected from the group comprising a nucleotide sequence coding for a signal peptide of MCP-1 or a derivative thereof having a nucleotide identity of at least 85 %, nucleotide sequence coding for a signal peptide of IL-6 or a derivative thereof having a nucleotide identity of at least 85 %, , a nucleotide sequence coding for a signal peptide of Ang-2 or a derivative thereof having a nucleotide identity of at least 85 %, and a nucleotide sequence coding for a signal peptide of Ang-1 or a derivative thereof having a nucleotide identity of at least 85 %.

In an eleventh embodiment of the first aspect, which is also an embodiment of the first, second, third, fourth, fifth, sixth, seventh, eighth, ninth and tenth embodiment of the first aspect, the cell a cellular function of which is restored and/or the cell in or on which a therapeutic effect is exercised is an endothelial cell, preferably a vascular endothelial cell, more preferably the vascular endothelial cell is a microvascular endothelial cell.

In 12^{th} embodiment of the first aspect, which is also an embodiment of the first, second, third, fourth, fifth, sixth, seventh, eighth, ninth, tenth and eleventh embodiment of the first aspect,wherein the cellular function is one which can be restored by an effector molecule having anti-permeability effect of endothelial cells, an anti-vascular leakage effect, an anti-apoptotic effect of endothelial cells or an anti-inflammatory effect of endothelial cells or an anti-stress response effect, preferably the effect is linked to or associated with the TIE-2 signalling pathway, VEGF-receptor pathway, NOTCH signalling pathway, PI3-kinase pathway, eNOS signalling pathway, sirtuin-dependent metabolic and energy homeostasis pathway, oxidative stress pathway, shear stress response pathway, ET-1 signal transduction pathway, NO-mediated signal transduction pathway, and mechanochemical transduction pathway.

More specifically, the problem underlying the present invention is solved in a second aspect, which is also a first embodiment of the second aspect, by a recombinant nucleic acid construct comprising in 5'-> 3' direction
- a 5' non-translated region,
- a coding region coding for an effector molecule, and
- a 3' non-translated region,
wherein the effector molecule is effective in restoring a cellular function of a cell, for use in a method for the treatment and/or prevention of a disease, wherein the effector molecule is expressed by an endothelial cell. In an embodiment and as preferably used herein restoring a cellular function comprises and, respectively, encompasses killing of a cell, more preferably killing of tumor endothelial cells and/or tumor cells; in another embodiment and as preferably used herein, restoring cellular function comprises and, respectively, encompasses neoangiogenesis, more preferably neoangiogenesis in case of vascular diseases.

In a second embodiment of the first aspect which is also an embodiment of the first embodiment of the second aspect, the treatment and/or prevention of a disease involves restoration of the cellular function of a cell.

More specifically, the problem underlying the present invention is solved in a third aspect, which is also a first embodiment of the third aspect, by a recombinant nucleic acid construct comprising in 5'-> 3' direction
- a 5' non-translated region,
- a coding region coding for an effector molecule, and
- a 3' non-translated region,
wherein the effector molecule is effective in restoring a cellular function of a cell, for use in a method for restoring the cellular function of a cell, wherein the effector molecule is expressed by an endothelial cell.

More specifically, the problem underlying the present invention is solved in a fourth aspect, which is also a first embodiment of the fourth aspect, by a recombinant nucleic acid construct comprising in 5'-> 3' direction
- a 5' non -translated region,
- a coding region coding for an effector molecule, and
- a 3' non-translated region,
wherein the effector molecule is effective in exercising a therapeutic effect in or on a cell, for use in a method for the treatment and/or prevention of a disease, wherein the effector molecule is expressed by an endothelial cell.

In a second embodiment of the fourth aspect which is also an embodiment of the first embodiment of the fourth aspect, the treatment and/or prevention of a disease involves restoration of a cellular function of a cell.

More specifically, the problem underlying the present invention is solved in a fifth aspect, which is also a first embodiment of the fifth aspect, by a recombinant nucleic acid construct comprising in 5'-> 3' direction
- a 5' non -translated region,
- a coding region coding for an effector molecule, and
- a 3' non-translated region,
wherein the effector molecule is effective in exercising a therapeutic effect in or on a cell, for use of in a method for restoring a cellular function of a cell, wherein the effector molecule is expressed by an endothelial cell.

In a third embodiment of the second aspect which is also an embodiment of the first and second embodiment of the second aspect, in a second embodiment of the third aspect which is also an embodiment of the first embodiment of the third aspect, in a third embodiment of the fourth aspect which is also an embodiment of the first and second embodiment of the fourth aspect, and in a second embodiment of the fifth aspect which is also an embodiment of the first embodiment of the fifth aspect, the effector molecule is expressed in the method by an endothelial cell.

In a fourth embodiment of the second aspect which is also an embodiment of the first, second and third embodiment of the second aspect, in a third embodiment of the third aspect which is also an embodiment of the first and the second embodiment of the third aspect, in a fourth embodiment of the fourth aspect which is also an embodiment of the first, second and third embodiment of the fourth aspect, and in a third embodiment of the fifth aspect which is also an embodiment of the first and second embodiment of the fifth aspect, the endothelial cell is a vascular endothelial cell, preferably the vascular endothelial cell is a microvascular endothelial cell.

In a fifth embodiment of the second aspect which is also an embodiment of the first, second, third and fourth embodiment of the second aspect, in a fourth embodiment of the third aspect which is also an embodiment of the first, second and third embodiment of the third aspect, in a fifth embodiment of the fourth aspect which is also an embodiment of the first, second, third and fourth embodiment of the fourth aspect, and in a fourth embodiment of the fifth aspect which is also an embodiment of the first, second and third embodiment of the fifth aspect, the endothelial cell is a non-dividing endothelial cell, a non-proliferating endothelial cell or a resting endothelial cell. In an embodiment thereof and as preferably used herein the endothelial is a vascular endothelial cell, a lymphatic endothelial cells, an endothelial stem cell (ESC) or an endothelial progenitor cells (EPC).

In a sixth embodiment of the second aspect which is also an embodiment of the first, second, third, fourth and fifth embodiment of the second aspect, in a fifth embodiment of the third aspect which is also an embodiment of the first, second, third and fourth embodiment of the third aspect, in a sixth embodiment of the fourth aspect which is also an embodiment of the first, second, third, fourth and fifth embodiment of the fourth aspect, and in a fifth embodiment of the fifth aspect which is also an embodiment of the first, second, third and fourth embodiment of the fifth aspect, the endothelial cell is a senescent endothelial cell.

In a seventh embodiment of the second aspect which is also an embodiment of the first, second, third, fourth, fifth and sixth embodiment of the second aspect, in a sixth embodiment of the third aspect which is also an embodiment of the first, second, third, fourth and fifth embodiment of the third aspect, in a seventh embodiment of the fourth aspect which is also an embodiment of the first, second, third, fourth, fifth and sixth embodiment of the fourth aspect, and in a sixth embodiment of the fifth aspect which is also an embodiment of the first, second, third, fourth and fifth embodiment of the fifth aspect, the endothelial cell is impaired by age and/or showing stress-related defects.

In an eighth embodiment of the second aspect which is also an embodiment of the first, second, third, fourth, fifth, sixth and seventh embodiment of the second aspect, in a seventh embodiment of the third aspect which is also an embodiment of the first, second, third, fourth, fifth and sixth embodiment of the third aspect, in an eighth embodiment of the fourth aspect which is also an embodiment of the first, second, third, fourth, fifth, sixth and seventh embodiment of the fourth aspect, and in a seventh embodiment of the fifth aspect which is also an embodiment of the first, second, third, fourth, fifth and sixth embodiment of the fifth aspect, the recombinant nucleic acid construct additionally comprises one element selected from the group comprising a cap structure, an IRES sequence, a further start codon providing sequence, a nucleic acid sequence coding for a signal peptide and a poly-A tail.

In a ninth embodiment of the second aspect which is also an embodiment of the first, second, third, fourth, fifth, sixth, seventh and eighth embodiment of the second aspect, in an eighth embodiment of the third aspect which is also an embodiment of the first, second, third, fourth, fifth, sixth and seventh embodiment of the third aspect, in a ninth embodiment of the fourth aspect which is also an embodiment of the first, second, third, fourth, fifth, sixth, seventh and eighth embodiment of the fourth aspect, and in an eighth embodiment of the fifth aspect which is also an embodiment of the first, second, third, fourth, fifth, sixth and seventh embodiment of the fifth aspect,
wherein the recombinant nucleic acid construct is one selected from the group comprising in 5'-> 3' direction
a)
   - a 5' non -translated region,
   - a coding region coding for an effector molecule,
   - a 3' non-translated region, and
   - a poly-A tail;
b)
   - a 5' non -translated region,
   - a further start codon providing sequence,
   - a coding region coding for an effector molecule,
   - a 3' non-translated region, and
   - a poly-A tail;
c)
   - a cap structure,
   - a 5' non -translated region,
   - a coding region coding for an effector molecule, and
   - a 3' non-translated region;
d)
   - a cap structure,
   - a 5' non -translated region,
   - a further start codon providing sequence,
   - a coding region coding for an effector molecule, and
   - a 3' non-translated region;
e)
   - an IRES sequence,
   - a 5' non -translated region,
   - a coding region coding for an effector molecule, and
   - a 3' non-translated region;
f)
   - an IRES sequence,
   - a 5' non -translated region,
   - a further start codon providing sequence,
   - a coding region coding for an effector molecule, and
   - a 3' non-translated region;
g)
   - a cap structure,
   - a 5' non -translated region,
   - a coding region coding for an effector molecule,
   - a 3' non-translated region, and
   - a poly-A tail;
h)
   - a cap structure,
   - a 5' non -translated region,
   - a further start codon providing sequence,
   - a coding region coding for an effector molecule,
   - a 3' non-translated region, and
   - a poly-A tail;
i)
   - an IRES sequence,
   - a 5' non -translated region,
   - a coding region coding for an effector molecule,
   - a 3' non-translated region, and
   - a poly-A tail;
j)
   - an IRES sequence,
   - a 5' non -translated region,
   - a further start codon providing sequence,
   - a coding region coding for an effector molecule,
   - a 3' non-translated region, and
   - a poly-A tail;
k)
   - a 5' non -translated region,
   - a nucleic acid sequence coding for a signal peptide,
   - a coding region coding for an effector molecule,
   - a 3' non-translated region, and
   - a poly-A tail;
l)
   - a 5' non -translated region,
   - a further start codon providing sequence,
   - a nucleic acid sequence coding for a signal peptide,
   - a coding region coding for an effector molecule,
   - a 3' non-translated region, and
   - a poly-A tail;
m)
   - a cap structure,
   - a 5' non -translated region,
   - a nucleic acid sequence coding for a signal peptide,
   - a coding region coding for an effector molecule, and
   - a 3' non-translated region;
   - a cap structure
   - a 5' non -translated region,
   - a further start codon providing sequence,
   - a nucleic acid sequence coding for a signal peptide,
   - a coding region coding for an effector molecule, and
   - a 3' non-translated region;
o)
   - an IRES sequence,
   - a 5' non -translated region,
   - a nucleic acid sequence coding for a signal peptide,
   - a coding region coding for an effector molecule, and
   - a 3' non-translated region;
p)
   - an IRES sequence,
   - a 5' non -translated region,
   - a further start codon providing sequence,
   - a nucleic acid sequence coding for a signal peptide,
   - a coding region coding for an effector molecule, and
   - a 3' non-translated region;
q)
   - a cap structure,
   - a 5' non -translated region,
   - a nucleic acid sequence coding for a signal peptide,
   - a coding region coding for an effector molecule,
   - a 3' non-translated region, and
   - a poly-A tail;
r)
   - a cap structure,
   - a 5' non -translated region,
   - a further start codon providing sequence,
   - a nucleic acid sequence coding for a signal peptide,
   - a coding region coding for an effector molecule,
   - a 3' non-translated region, and
   - a poly-A tail;
s)
   - an IRES sequence,
   - a 5' non -translated region,
   - a nucleic acid sequence coding for a signal peptide,
   - a coding region coding for an effector molecule,
   - a 3' non-translated region, and
   - a poly-A tail; and
t)
   - an IRES sequence,
   - a 5' non -translated region,
   - a further start codon providing sequence,
   - a nucleic acid sequence coding for a signal peptide,
   - a coding region coding for an effector molecule,
   - a 3' non-translated region, and
   - a poly-A tail.

In a tenth embodiment of the second aspect which is also an embodiment of the first, second, third, fourth, fifth, sixth, seventh, eighth and ninth embodiment of the second aspect, in an ninth embodiment of the third aspect which is also an embodiment of the first, second, third, fourth, fifth, sixth, seventh and eighth embodiment of the third aspect, in a tenth embodiment of the fourth aspect which is also an embodiment of the first, second, third, fourth, fifth, sixth, seventh, eighth and ninth embodiment of the fourth aspect, and in an ninth embodiment of the fifth aspect which is also an embodiment of the first, second, third, fourth, fifth, sixth, seventh and eighth embodiment of the fifth aspect, the recombinant nucleic acid comprises in 5' -> 3' direction
- a cap structure,
- a 5' non -translated region,
- a nucleic acid sequence coding for a signal peptide,
- a coding region coding for an effector molecule,
- a 3' non-translated region, and
- a poly-A tail.

In an eleventh embodiment of the second aspect which is also an embodiment of the first, second, third, fourth, fifth, sixth, seventh, eighth, ninth and tenth embodiment of the second aspect, in a tenth embodiment of the third aspect which is also an embodiment of the first, second, third, fourth, fifth, sixth, seventh, eighth and ninth embodiment of the third aspect, in an eleventh tenth embodiment of the fourth aspect which is also an embodiment of the first, second, third, fourth, fifth, sixth, seventh, eighth, ninth and tenth embodiment of the fourth aspect, and in a tenth embodiment of the fifth aspect which is also an embodiment of the first, second, third, fourth, fifth, sixth, seventh, eighth and ninth embodiment of the fifth aspect, the recombinant nucleic acid comprises in 5' -> 3' direction
- an IRES sequence,
- a 5' non -translated region,
- a nucleic acid sequence coding for a signal peptide,
- a coding region coding for an effector molecule,
- a 3' non-translated region, and
- a poly-A tail.

In a 12th embodiment of the second aspect which is also an embodiment of the first, second, third, fourth, fifth, sixth, seventh, eighth, ninth, tenth and eleventh embodiment of the second aspect, in an eleventh embodiment of the third aspect which is also an embodiment of the first, second, third, fourth, fifth, sixth, seventh, eighth, ninth and tenth embodiment of the third aspect, in a 12^{th} embodiment of the fourth aspect which is also an embodiment of the first, second, third, fourth, fifth, sixth, seventh, eighth, ninth, tenth and eleventh embodiment of the fourth aspect, and in an eleventh embodiment of the fifth aspect which is also an embodiment of the first, second, third, fourth, fifth, sixth, seventh, eighth, ninth and tenth embodiment of the fifth aspect, recombinant nucleic acid comprises in 5' -> 3' direction
- a cap structure,
- a 5' non -translated region,
- a further start codon providing sequence,
- a nucleic acid sequence coding for a signal peptide,
- a coding region coding for an effector molecule,
- a 3' non-translated region, and
- a poly-A tail.

In a 13^{th} embodiment of the second aspect which is also an embodiment of the first, second, third, fourth, fifth, sixth, seventh, eighth, ninth, tenth, eleventh and 12^{th} embodiment of the second aspect, in a 12^{th} embodiment of the third aspect which is also an embodiment of the first, second, third, fourth, fifth, sixth, seventh, eighth, ninth, tenth and eleventh embodiment of the third aspect, in a 13^{th} embodiment of the fourth aspect which is also an embodiment of the first, second, third, fourth, fifth, sixth, seventh, eighth, ninth, tenth, eleventh and 12^{th} embodiment of the fourth aspect, and in an 12^{th} embodiment of the fifth aspect which is also an embodiment of the first, second, third, fourth, fifth, sixth, seventh, eighth, ninth, tenth and eleventh embodiment of the fifth aspect, the recombinant nucleic acid comprises in 5' -> 3' direction
- an IRES sequence,
- a 5' non -translated region,
- a further start codon providing sequence,
- a nucleic acid sequence coding for a signal peptide,
- a coding region coding for an effector molecule,
- a 3' non-translated region, and
- a poly-A tail.

In a 14^{th} embodiment of the second aspect which is also an embodiment of the first, second, third, fourth, fifth, sixth, seventh, eighth, ninth, tenth, eleventh, 12^{th} and 13^{th} embodiment of the second aspect, in a 13^{th} embodiment of the third aspect which is also an embodiment of the first, second, third, fourth, fifth, sixth, seventh, eighth, ninth, tenth, eleventh and 12^{th} embodiment of the third aspect, in a 14^{th} embodiment of the fourth aspect which is also an embodiment of the first, second, third, fourth, fifth, sixth, seventh, eighth, ninth, tenth, eleventh, 12^{th} and 13^{th} embodiment of the fourth aspect, and in an 13^{th} embodiment of the fifth aspect which is also an embodiment of the first, second, third, fourth, fifth, sixth, seventh, eighth, ninth, tenth, eleventh and 12^{th} embodiment of the fifth aspect, the further start codon providing sequence is in-frame with the coding region coding for an effector molecule.

In a 15^{th} embodiment of the second aspect which is also an embodiment of the first, second, third, fourth, fifth, sixth, seventh, eighth, ninth, tenth, eleventh, 12^{th}, 13^{th} and 14^{th} embodiment of the second aspect, in a 14^{th} embodiment of the third aspect which is also an embodiment of the first, second, third, fourth, fifth, sixth, seventh, eighth, ninth, tenth, eleventh, 12^{th} and 13^{th} embodiment of the third aspect, in a 15^{th} embodiment of the fourth aspect which is also an embodiment of the first, second, third, fourth, fifth, sixth, seventh, eighth, ninth, tenth, eleventh, 12^{th}, 13^{th} and 14^{th} embodiment of the fourth aspect, and in an 14^{th} embodiment of the fifth aspect which is also an embodiment of the first, second, third, fourth, fifth, sixth, seventh, eighth, ninth, tenth, eleventh 12^{th} and 13^{th} embodiment of the fifth aspect, the 5' non -translated region is a 5' UTR.

In a 16^{th} embodiment of the second aspect which is also an embodiment of the first, second, third, fourth, fifth, sixth, seventh, eighth, ninth, tenth, eleventh, 12^{th}, 13^{th}, 14^{th} and 15^{th} embodiment of the second aspect, in a 15^{th} embodiment of the third aspect which is also an embodiment of the first, second, third, fourth, fifth, sixth, seventh, eighth, ninth, tenth, eleventh, 12^{th}, 13^{th} and 14^{th} embodiment of the third aspect, in a 16^{th} embodiment of the fourth aspect which is also an embodiment of the first, second, third, fourth, fifth, sixth, seventh, eighth, ninth, tenth, eleventh, 12^{th}, 13^{th}, 14^{th} and 15^{th} embodiment of the fourth aspect, and in an 15^{th} embodiment of the fifth aspect which is also an embodiment of the first, second, third, fourth, fifth, sixth, seventh, eighth, ninth, tenth, eleventh 12^{th}, 13^{th} and 14^{th} embodiment of the fifth aspect, the 5' non-translated region is from a eukaryotic organism, preferably a mammalian organism, more preferably a mammalian organism selected from the group comprising human, non-human primate such as cynomolgus, chimpanzee rhesus monkey, rat and mouse. In an alternative embodiment the 5' non-translated region is from a viral gene, preferably the virus is a virus capable of infecting a eukaryotic organism.

In a 17^{th} embodiment of the second aspect which is also an embodiment of the first, second, third, fourth, fifth, sixth, seventh, eighth, ninth, tenth, eleventh, 12^{th}, 13^{th}, 14^{th}, 15^{th} and 16^{th} embodiment of the second aspect, in a 16^{th} embodiment of the third aspect which is also an embodiment of the first, second, third, fourth, fifth, sixth, seventh, eighth, ninth, tenth, eleventh, 12^{th}, 13^{th}, 14^{th} and 15^{th} embodiment of the third aspect, in a 17^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th} and 16^{th} embodiment of the fourth aspect, and in an 16^{th} embodiment of the fifth aspect which is also an embodiment of the first, second, third, fourth, fifth, sixth, seventh, eighth, ninth, tenth, eleventh 12^{th}, 13^{th}, 14^{th} and 15^{th} embodiment of the fifth aspect, the 5' UTR is selected from the group comprising a 5' UTR of a gene coding for MCP-1 or a derivative thereof having a nucleotide identity of at least 85 %, a 5' UTR of a gene coding for Ang-2 or a derivative thereof having a nucleotide identity of at least 85 %, a 5' UTR of a gene coding for RPL12s.c. or a derivative thereof having a nucleotide identity of at least 85 %, a 5' UTR of a gene coding for HSP70 or a derivative thereof having a nucleotide identity of at least 85 %, a 5' UTR of a gene coding for H3.3. or a derivative thereof having a nucleotide identity of at least 85 %, and a 5' UTR of a gene coding for Galectin-9 or a derivative thereof having a nucleotide identity of at least 85 %.

In an 18^{th} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th} and 17^{th} embodiment of the second aspect, in a 17^{th} embodiment of the third aspect which is also an embodiment of the first, second, third, fourth, fifth, sixth, seventh, eighth, ninth, tenth, eleventh, 12^{th}, 13^{th}, 14^{th}, 15^{th} and 16^{th} embodiment of the third aspect, in an 18^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th} and 17^{th} embodiment of the fourth aspect, and in an 17^{th} embodiment of the fifth aspect which is also an embodiment of the first, second, third, fourth, fifth, sixth, seventh, eighth, ninth, tenth, eleventh 12^{th}, 13^{th}, 14^{th} and 15^{th}, 16^{th} embodiment of the fifth aspect, the 3' non-translated region is a 3'-UTR.

In a 19^{th} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th} and 18^{th} embodiment of the second aspect, in an 18^{th} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th} and 17^{th} embodiment of the third aspect, in a 19^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th} , 17^{th} and 18^{th} embodiment of the fourth aspect, and in an 18^{th} embodiment of the fifth aspect which is also an embodiment of the first, second, third, fourth, fifth, sixth, seventh, eighth, ninth, tenth, eleventh 12^{th}, 13^{th}, 14^{th} and 15^{th}, 16^{th}, 17^{th} embodiment of the fifth aspect, the 3' non-translated region is from a eukaryotic organism, preferably a mammalian organism, more preferably a mammalian organism selected from the group comprising human, non-human primate such as cynomolgus, chimpanzee rhesus monkey, rat and mouse. In an alternative embodiment the 5' non-translated region is from a viral gene, preferably the virus is a virus capable of infecting a eukaryotic organism.

In a 20^{th} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th} and 19^{th} embodiment of the second aspect, in a 19^{th} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th} and 18^{th} embodiment of the third aspect, in a 20^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th} , 17^{th}, 18^{th} and 19^{th} embodiment of the fourth aspect, and in a 19^{th} embodiment of the fifth aspect which is also an embodiment of the first, second, third, fourth, fifth, sixth, seventh, eighth, ninth, tenth, eleventh 12^{th}, 13^{th}, 14^{th}, 15^{th}, 16^{th}, 17^{th} and 18^{th} embodiment of the fifth aspect, the 3' UTR is selected from the group comprising a 3' UTR of a gene coding for MCP-1 or a derivative thereof having a nucleotide identity of at least 85 %, a 3' UTR of a gene coding for vWF or a derivative thereof having a nucleotide identity of at least 85 %, a 3' UTR of a gene coding for RPL12s.c. or a derivative thereof having a nucleotide identity of at least 85 %, a 3' UTR of a gene coding for HSP70 or a derivative thereof having a nucleotide identity of at least 85 %, and a 3' UTR of a gene coding for H3.3. or a derivative thereof having a nucleotide identity of at least 85 %.

In a 21^{st} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th} and 20^{th} embodiment of the second aspect, in a 20^{th} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th} and 19^{th} embodiment of the third aspect, in a 21^{st} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th} , 17^{th}, 18^{th} 19^{th} and 20^{th} embodiment of the fourth aspect, and in a 20^{th} embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th} and 19^{th} embodiment of the fifth aspect,
a) the 5' UTR is a 5' UTR of a gene coding for HSP70 or a derivative thereof having a nucleotide identity of at least 85 %, and wherein the 3' UTR is selected from the group comprising a 3' UTR of a gene coding for HSP70 or a derivative thereof having a nucleotide identity of at least 85 %, a 3' UTR of a gene coding for RPL12s.c. or a derivative thereof having a nucleotide identity of at least 85 %, a 3' UTR of a gene coding for vWF or a derivative thereof having a nucleotide identity of at least 85 % and a 3' UTR of a gene coding for MCP-1 or a derivative thereof having a nucleotide identity of at least 85 %,
b) the 5' UTR is a 5' UTR of a gene coding for RPL12s.c. or a derivative thereof having a nucleotide identity of at least 85 %, and wherein the 3' UTR is selected from the group comprising a 3' UTR of a gene coding for HSP70 or a derivative thereof having a nucleotide identity of at least 85 %, a 3' UTR of a gene coding for RPL12s.c. or a derivative thereof having a nucleotide identity of at least 85 %, a 3' UTR of a gene coding for vWF or a derivative thereof having a nucleotide identity of at least 85 % and a 3' UTR of a gene coding for MCP-1 or a derivative thereof having a nucleotide identity of at least 85 %,
c) the 5' UTR is a 5' UTR of a gene coding for ANG-2 or a derivative thereof having a nucleotide identity of at least 85 %, and wherein the 3' UTR is selected from the group comprising a 3' UTR of a gene coding for HSP70 or a derivative thereof having a nucleotide identity of at least 85 %, a 3' UTR of a gene coding for RPL12s.c. or a derivative thereof having a nucleotide identity of at least 85 %, a 3' UTR of a gene coding for vWF or a derivative thereof having a nucleotide identity of at least 85 % and a 3' UTR of a gene coding for MCP-1 or a derivative thereof having a nucleotide identity of at least 85 %, or
d) the 5' UTR is a 5' UTR of a gene coding for MCP-1 or a derivative thereof having a nucleotide identity of at least 85 %, and wherein the 3' UTR is selected from the group comprising a 3' UTR of a gene coding for HSP70 or a derivative thereof having a nucleotide identity of at least 85 %, a 3' UTR of a gene coding for RPL12s.c. or a derivative thereof having a nucleotide identity of at least 85 %, a 3' UTR of a gene coding for vWF or a derivative thereof having a nucleotide identity of at least 85 % and a 3' UTR of a gene coding for MCP-1 or a derivative thereof having a nucleotide identity of at least 85 %.

In a 22^{nd} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th} and 21^{st} embodiment of the second aspect, in a 21^{st} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th} and 20^{th} embodiment of the third aspect, in a 22^{nd} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th} and 21^{st} embodiment of the fourth aspect, and in a 21^{st} embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th}, 19^{th} and 20^{th} embodiment of the fifth aspect,
a) 3'UTR is a 3' UTR of a gene coding for HSP70 or a derivative thereof having a nucleotide identity of at least 85 % and the 5' UTR is selected from the group comprising a 5' UTR of a gene coding for HSP70 or a derivative thereof having a nucleotide identity of at least 85 % a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %,
b) 3'UTR is a 3' UTR of a gene coding for RPL12s.c. or a derivative thereof having a nucleotide identity of at least 85 % and the 5' UTR is selected from the group comprising a 5' UTR of a gene coding for HSP70 or a derivative thereof having a nucleotide identity of at least 85 % a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %,
c) 3'UTR is a 3' UTR of a gene coding for vWF or a derivative thereof having a nucleotide identity of at least 85 % and the 5' UTR is selected from the group comprising a 5' UTR of a gene coding for HSP70 or a derivative thereof having a nucleotide identity of at least 85 % a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %, or
d) 3'UTR is a 3' UTR of a gene coding for MCP-1 or a derivative thereof having a nucleotide identity of at least 85 % and the 5' UTR is selected from the group comprising a 5' UTR of a gene coding for HSP70 or a derivative thereof having a nucleotide identity of at least 85 %, a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %.

In a 23^{rd} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st} and 22^{nd} embodiment of the second aspect, in a 22^{nd} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th} and 21^{st} embodiment of the third aspect, in a 23^{rd} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st} and 22^{nd} embodiment of the fourth aspect, and in a 22^{nd} embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th}, 19^{th}, 20^{th} and 21^{st} embodiment of the fifth aspect, the construct is one selected from the group comprising
a construct, wherein the 5' UTR is a 5' UTR of a gene coding for MCP-1 or a derivative thereof having a nucleotide identity of at least 85 %, and the 3' UTR is a 3' UTR of a gene coding for MCP-1 or a derivative thereof having a nucleotide identity of at least 85 %, a construct, wherein the 5' UTR is a 5' UTR of a gene coding for ANG-2 or a derivative thereof having a nucleotide identity of at least 85 %, and the 3' UTR is a 3' UTR of a gene coding for vWF or a derivative thereof having a nucleotide identity of at least 85 %, a construct, wherein the 5' UTR is a 5' UTR of a gene coding for RPL12s.c. or a derivative thereof having a nucleotide identity of at least 85 %, and the 3' UTR is a 3' UTR of a gene coding for RPL12s.c. or a derivative thereof having a nucleotide identity of at least 85 %,
a construct, wherein the 5' UTR is a 5' UTR of a gene coding for HSP70 or a derivative thereof having a nucleotide identity of at least 85 %, and the 3' UTR is a 3' UTR of a gene coding for HSP70 or a derivative thereof having a nucleotide identity of at least 85 %,
a construct, wherein the 5' UTR is a 5' UTR of a gene coding for H3.3. or a derivative thereof having a nucleotide identity of at least 85 %, and the 3' UTR is a 3' UTR of a gene coding for H3.3. or a derivative thereof having a nucleotide identity of at least 85 %, or
a construct, wherein the 5' UTR is a 5' UTR of a gene coding for Galectin 9 or a derivative thereof having a nucleotide identity of at least 85 %, and the 3' UTR is a 3' UTR of a gene coding for Galectin 9 or a derivative thereof having a nucleotide identity of at least 85 %.

In a 24th embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd} and 23^{rd} embodiment of the second aspect, in a 23^{rd} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st} and 22^{nd} embodiment of the third aspect, in a 24^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th} , 17^{th}, 18^{th} , 19^{th}, 20^{th}, 21^{st} , 22^{nd} and 23^{rd} embodiment of the fourth aspect, and in a 23^{rd} embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th}, 19^{th}, 20^{th}, 21^{st} and 22^{nd} embodiment of the fifth aspect, the construct comprises a poly-A tail.

In a 25^{th} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd} and 23^{rd} and 24^{th} embodiment of the second aspect, in a 24^{th} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd} and 23^{rd} embodiment of the third aspect, in a 25^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th} , 17^{th}, 18^{th} , 19^{th}, 20^{th}, 21^{st}, 22^{nd} 23^{rd} and 24^{th} embodiment of the fourth aspect, and in a 24^{th} embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th}, 19^{th}, 20^{th}, 21^{st}, 22^{nd} and 23^{rd} embodiment of the fifth aspect, the construct comprises CAP structure.

In a 26^{th} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd} and 23^{rd}, 24^{th} and 25^{th} embodiment of the second aspect, in a 25^{th} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd}, 23^{rd} and 24^{th} embodiment of the third aspect, in a 26^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st} , 22^{nd}, 23^{rd}, 24^{th} and 25^{th} embodiment of the fourth aspect, and in a 25^{th} embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th}, 19^{th}, 20^{th}, 21^{st}, 22^{nd} 23^{rd} and 24^{th} embodiment of the fifth aspect, the construct comprises one or more IRESs (internal ribosomal entry sites) sequences. In a preferred embodiment, the IRES sequences replaced the Cap structure.

In a 27^{th} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd} and 23^{rd}, 24^{th}, 25^{th} and 26^{th} embodiment of the second aspect, in a 26^{th} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd}, 23^{rd}, 24^{th} and 25^{th} embodiment of the third aspect, in a 27^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th} and 26^{th} embodiment of the fourth aspect, and in a 26^{th} embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th}, 19^{th}, 20^{th}, 21^{st}, 22^{nd}, 23^{rd}, 24^{th} and 25^{th} embodiment of the fifth aspect, the construct comprises nucleic acid sequence coding for a signal peptide.

In a 28^{th} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd} and 23^{rd}, 24^{th}, 25^{th}, 26^{th} and 27^{th} embodiment of the second aspect, in a 27^{th} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th} and 26^{th} embodiment of the third aspect, in a 28^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st} , 22^{nd}, 23^{rd}, 24^{th}, 25^{th}, 26^{th} and 27^{th} embodiment of the fourth aspect, and in a 27^{th} embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th}, 19^{th}, 20^{th}, 21^{st}, 22^{nd}, 23^{rd}, 24^{th}, 25^{th} and 26^{th} embodiment of the fifth aspect, the signal peptide allows secretion of the effector molecule.

In a 29^{th} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd} and 23^{rd}, 24^{th}, 25^{th}, 26^{th} , 27^{th} and 28^{th} embodiment of the second aspect, in a 28^{th} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th}, 26^{th} and 27^{th} embodiment of the third aspect, in a 29^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th} , 17^{th}, 18^{th} , 19^{th}, 20^{th}, 21^{st} , 22^{nd}, 23^{rd}, 24^{th}, 25^{th}, 26^{th}, 27^{th} and 28^{th} embodiment of the fourth aspect, and in a 28^{th} embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th}, 19^{th}, 20^{th}, 21^{st}, 22^{nd}, 23^{rd}, 24^{th}, 25^{th}, 26^{th} and 27^{th} embodiment of the fifth aspect, the nucleotide sequence coding for a signal peptide is selected from the group comprising a nucleotide sequence coding for a signal peptide of MCP-1 or a derivative thereof having a nucleotide identity of at least 85 %, nucleotide sequence coding for a signal peptide of IL-6 or a derivative thereof having a nucleotide identity of at least 85 %,, a nucleotide sequence coding for a signal peptide of Ang-2 or a derivative thereof having a nucleotide identity of at least 85 %, and a nucleotide sequence coding for a signal peptide of Ang-1 or a derivative thereof having a nucleotide identity of at least 85 %.

In an embodiment of the first, second, third, fourth and fifth aspect, including any embodiment thereof, the cell a cellular function of which is restored and/or the cell in or on which a therapeutic effect is exercised is an endothelial cell, preferably a vascular endothelial cell, more preferably the vascular endothelial cell is a microvascular endothelial cell.

In an embodiment of the first, second, third, fourth and fifth aspect, including any embodiment thereof, the endothelial cell is a non-dividing endothelial cell, a non-proliferating endothelial cell or a resting endothelial cell.

In an embodiment of the first, second, third, fourth and fifth aspect, including any embodiment thereof, the endothelial cell is an endothelial cell stimulated by an inflammatory stimulus.

In an embodiment of the first, second, third, fourth and fifth aspect, including any embodiment thereof, the endothelial cell is a senescent endothelial cell.

In an embodiment of the first, second, third, fourth and fifth aspect, including any embodiment thereof, the endothelial cell is impaired by age and/or showing stress-related defects.

In an embodiment of the first, second, third, fourth and fifth aspect, including any embodiment thereof, the cellular function is one which can be restored by an effector molecule having anti-permeability effect of endothelial cells, an anti-vascular leakage effect, an anti-apoptotic effect of endothelial cells or an anti-inflammatory effect of endothelial cells or an anti-stress response effect. In an embodiment thereof, the effector molecule stimulates vessel survival, inhibits regression, inhibits apoptosis, stimulates migration, stimulates remodelling, stimulates angiogenesis, stimulates tube-formation/invasion, stimulates proliferation, inhibits leucocyte adhesion, inhibits adhesion molecule expression, inhibits tissue factor expression, inhibits NFKappaB activity or promotes monolayer integrity.

In an embodiment of the first, second, third, fourth and fifth aspect, including any embodiment thereof, the effect is linked to or associated with the TIE-2 signalling pathway, VEGF-receptor pathway, NOTCH signalling pathway, PI3-kinase pathway, eNOS signalling pathway, sirtuin-dependent metabolic and energy homeostasis pathway, oxidative stress pathway, shear stress response pathway, ET-1 signal transduction pathway, NO-mediated signal transduction pathway, and mechanochemical transduction pathway, preferably the pathway is selected from the group comprising Ang/TIE2 signalling pathway, VEGF/VEGF receptor pathway and Notch/Notch ligand pathway.

In an embodiment of the first, second, third, fourth and fifth aspect, including any embodiment thereof, the effector molecule is an element of a pathway, wherein the pathway is selected from the group comprising the TIE-2 signalling pathway, VEGF-Receptor pathway, NOTCH signalling pathway, PI3-kinase pathway, eNOS signalling pathway, sirtuin-dependent metabolic and energy homeostasis pathway, oxidative stress pathway, shear stress response pathway ET-1 signal transduction pathway, NO-mediated signal transduction pathway, and mechanochemical transduction pathway.

In an embodiment of the first, second, third, fourth and fifth aspect, including any embodiment thereof, the effector molecule is selected from the group comprising Ang-1, Ang-4, COMP-Ang-1, hCOMP-Ang-1, COMP-Ang-1, COMP-ANG-2, Tie2 receptor, Tie1 receptor, PI3-kinase, preferably constitutive active PI3-kinase, hyperactive Tie2 receptor (e.g. R849W), VE-cadherin, GRB2, GRB7, GRB14, GRB7, IQGAP1, RAC1, RAP1, DOK2, ABIN1 and ABIN2, KLF2 (Krueppel-like factor 2), alpha5 beta1 integrin, CD73, Akt 1, Akt 2 and Akt 3.

In an embodiment of the first, second, third, fourth and fifth aspect, including any embodiment thereof, the cellular function is vascular leakage and, preferably, the pathway is the TIE-2 pathway.

In an embodiment of the first, second, third, fourth and fifth aspect, including any embodiment thereof, the effector molecule is selected from the group comprising VEGF, VEGF-A, VEGF-B, PDGF, bFGF, Sirtuin, eNOS, RAS and c-MYC.

In an embodiment of the first, second, third, fourth and fifth aspect, including any embodiment thereof, wherein the effector molecule restores vascular regeneration or provides for vascular regeneration, preferably vascular endothelial vascular regeneration

In an embodiment of the first, second, third, fourth and fifth aspect, including any embodiment thereof, the cell a cellular function of which is restored and/or the cell in or on which a therapeutic effect is exercised is a non-endothelial cell.

In an embodiment of the first, second, third, fourth and fifth aspect, including any embodiment thereof, the non-endothelial cell is selected from the group comprising epithelial cells, aveolar macrophages, Alveolar type I cells, alveolar type II cells, alveolar macrophages, Pneumocytes, lung epithelial cells, hematopoietic cells, bone marrow cells, bone cells, stem cells, mesenchymal cells, neural cells, glia cells, neuron cells, Astrocytes, cells of the peripheral nervous system, cardiac cells, adipocytes, vascular smooth muscle cells, cardiomyocytes, skeletal muscle cells, beta cells, pituitary cells, synovial lining cells, ovarian cells, testicular cells, B cells, T cells, reticulocytes, leukocytes, granulocytes, macrophages, neutrophils, antigen presenting cells (dendritic cells), fibroblasts, hepatocytes, tumor cells and combination thereof.

In an embodiment of the first, second, third, fourth and fifth aspect, including any embodiment thereof, the effector molecule is selected from the group comprising a tumor death ligand, a factor involved in hematopoiesis and blood clotting, a stem cell factor, a growth factors and a cytokine.

In an embodiment of the first, second, third, fourth and fifth aspect, including any embodiment thereof, the tumor death ligand is selected from the group comprising IL-12, members of the TNF gene superfamily of death ligand proteins such as Apo2L/TRAIL, IL13, IL10, IL8, IL2, interferon beta, secreted frizzled-related protein (SFRP) 1, SFRP 2, SFRP 3, SFRP 4 and SFRP5).

In an embodiment of the first, second, third, fourth and fifth aspect, including any embodiment thereof, wherein the factor involved in hematopoiesis and blood clotting is selected from the group comprising erythropoietin, Factor VII, Factor VIII, Factor IX and heparan-N-sulfatase.

In an embodiment of the first, second, third, fourth and fifth aspect, including any embodiment thereof, the stem cell factor is selected from the group comprising Oct4, Sox2, Klf4 and c-Myc.

In an embodiment of the first, second, third, fourth and fifth aspect, including any embodiment thereof, the growth factor and cytokine is selected from the group comprising Adrenomedullin, Angiopoietin family, Autocrine motility factor, Bone morphogenetic proteins, Ciliary neurotrophic factor (CNTF), Leukemia inhibitory factor (LIF), Interleukin-6 (IL-6), Macrophage colony-stimulating factor (m-CSF), Granulocyte colony-stimulating factor (G-CSF), Granulocyte macrophage colony-stimulating factor (GM-CSF), Epidermal growth factor (EGF), Ephrins , Erythropoietin (EPO), Fibroblast growth factor family (FGF1-23), Somatotrophin , GDNF family of ligands (such as Glial cell line-derived neurotrophic factor (GDNF), Neurturin, Persephin, Artemin), Growth differentiation factor-9 (GDF9),Hepatocyte growth factor (HGF),Hepatoma-derived growth factor (HDGF), Insulin, Insulin-like growth factors -1 and-2 (IGF-1) (IGF-2), Interleukins 1-7, Keratinocyte growth factor (KGF), Migration-stimulating factor (MSF), Macrophage-stimulating protein (MSP), also known as hepato-cyte growth factor-like protein (HGFLP), Myostatin (GDF-8), Neuregulins 1-4 (NRG1-4), Neurotrophins (Brain-derived neurotrophic factor (BDNF), Nerve growth factor (NGF), Neu-rotrophin-3 (NT-3), Neurotrophin-4 (NT-4)), Placental growth factor (PGF), Platelet-derived growth factor (PDGF), Renalase (RNLS) - Anti-apoptotic survival factor, T-cell growth fac-tor (TCGF), Thrombopoietin (TPO), Transforming growth factor alpha (TGF-α), Transforming growth factor beta (TGF-β), Tumor necrosis factor-alpha (TNF-α), Vascular endothelial growth factor family (VEGF), Wnt Signaling Pathway signaling glycoproteins (WNT1, WNT2, WNT2B, WNT3, WNT3A, WNT4, WNT5A, WNT5B, WNT6, WNT7A, WNT7B, WNT8A, WNT8B, WNT9A, WNT9B, WNT10A, WNT10B, WNT11, WNT16), Bone morphogenetic protein (BMP), Erythropoietin (EPO), Granulocyte colony-stimulating factor (G-CSF), Granulocyte macrophage colony-stimulating factor (GM-CSF), Interferon alfa, Interferon beta , Interleukin 2 (IL-2), Interleukin 11 (IL-11), Interferon gamma, IL1, IL3, IL4, IL5, IL6, IL7, IL8, IL9, IL10, IL12,IL13, IL14, IL15, IL16, IL17, IL18, IL19, IL20, IL21, IL22, IL23,IL24, IL25, IL26, IL27, IL28, IL29, IL30, IL31, IL32,. IL33, IL35 and IL36.

In an embodiment of the first, second, third, fourth and fifth aspect, including any embodiment thereof, the disease is characterized by or caused by vascular leakage, preferably leakage of vascular endothelial cells, more preferably leakage of micro vascular endothelial cells.

In an embodiment of the first, second, third, fourth and fifth aspect, including any embodiment thereof, a subject suffering from the disease shows inflammation in the lung.

In an embodiment of the first, second, third, fourth and fifth aspect, including any embodiment thereof, the disease wherein the disease is selected from the group comprising pneumonia, sepsis and trauma.

In an embodiment of the first, second, third, fourth and fifth aspect, including any embodiment thereof, a subject suffering from the disease shows acute respiratory distress syndrome (ARDS).

In an embodiment of the first, second, third, fourth and fifth aspect, including any embodiment thereof, disease is pneumonia, preferably pneumonia selected from the group comprising severe community-acquired pneumonia (sCAP), community acquired pneumonia (CAP), hospital-acquired pneumonia (HAP).

In an embodiment of the first, second, third, fourth and fifth aspect, including any embodiment thereof, the disease is characterized by or shows lung damage.

In an embodiment of the first, second, third, fourth and fifth aspect, including any embodiment thereof, lung damage is immediate lung damage.

In an embodiment of the first, second, third, fourth and fifth aspect, including any embodiment thereof, immediate lung damage results from inhalation of toxic gases, toxic lung edema, lung infection of a virus or bacterium, aspiration of stomach contents, aspiration of fresh water, aspiration of salt water, pulmonary contusion, fat embolism, amniotic fluid embolism and inhalation of hyperbaric oxygen.

In an embodiment of the first, second, third, fourth and fifth aspect, including any embodiment thereof, lung damage is indirect lung damage.

In an embodiment of the first, second, third, fourth and fifth aspect, including any embodiment thereof, indirect lung damage results from sepsis, bacteremia, endotoxinemia, severe trauma, polytrauma including shock, burns, pancreatitis, malaria tropica, drugs and immunosuppression, chronic alcohol abuse, chronic pulmonary diseases, low pH of serum.

In an embodiment of the first, second, third, fourth and fifth aspect, including any embodiment thereof, the disease or effect is vascular regeneration or longevity, wherein preferably the effector molecule is selected from the group comprising VEGF, VEGF-A, VEGF-B, PDGF, bFGF, Sirtuin (Sirt 1), PPAR-gamma, AMPK, eNOS, RAS, c-MYC, FOXO3, PI3 kinase *, Akt, Akt*, Adenosine A1 receptor, Adenosine A2A receptor, Adeno-sine A2B receptor, Telomerase, Yamanaka factors (Oct4, sox2, klf4, c-My).

In an embodiment of the first, second, third, fourth and fifth aspect, including any embodiment thereof, the effector molecule is an antiapoptotic factor, preferably protecting normal, i.e. non-diseased endothelium, wherein the antiapoptotic factor is preferably selected ffrom the group comprising PTEN, survivin, IAP, cIAP2 and XIAP, neuroglobin (binds cytochrome c), prosurvival pro-teins Bcl-2, BCL-xl, BCL-w, mcl-1 A1, NR-13, BHRF1, LMW5-HL, ORF16, KS-BCl-2, E1b-19K and P53.

In an embodiment of the first, second, third, fourth and fifth aspect, including any embodiment thereof, the effector molecule is an apoptotic factor, preferably targeting tumor endothelium, wherein the apoptotic factor is preferably selected from the group comprising PTEN, survivin, IAP, cIAP2 and XIAP, neuroglobin (binds cytochrome c), prosurvival proteins Bcl-2, BCL-xl, BCL-w, mcl-1 A1, NR-13, BHRF1, LMW5-HL, ORF16, KS-BCl-2, E1b-19K, P53.

In an embodiment of the first, second, third, fourth and fifth aspect, including any embodiment thereof, the effector molecule is a tumor death ligand, preferably the tumor death ligand is proapoptotic or anti-angiogenic, more preferably the tumor death ligand is selected from the group comprising Members of the TNF gene superfamily of death ligand proteins such as TNF-alpha, Fas-L, Apo2L/TRAIL, IL-12, IL13, IL10, IL8, IL2, interferon beta, secreted frizzled-related protein (SFRP) family (SFRP1, 2, 3, 4, 5), ELTD1, TGF-β, TNF-a, IL-6, PTEN, p53 and other tumor suppressor genes with proapoptotic function, Thrombospondin C, TIMP-1, TIMP-2, interferon-alpha, interferon-beta, interferon-gamma, Ang-1 and derivatives with TIE-2 agonistic function (see claim xx), cytochrome c, BH3-only proteins, pro- apoptotic Bcl-2 proteins Bad, Bid, Bax, Bak and Bim, Apaf1, procaspase-8, 9 10, caspases 2, 3, 6, 7, 8, 9 and 10.

In an embodiment of the first, second, third, fourth and fifth aspect, including any embodiment thereof, the effector molecule is anti-inflammatory, preferably the effector molecule is selected from the group comprising IkappaB and derivative with mutated phosphorylation sites, PI3 kinase and AKT kinases and hyperactive forms.

In an embodiment of the first, second, third, fourth and fifth aspect, including any embodiment thereof, the effector molecule is active in blood coagulation and/or hematopoiesis, preferably the effector molecule is selected from the group comprising erythropoietin, factor VII, factor VIII, factor IX and heparan-N-sulfatase.

In an embodiment of the first, second, third, fourth and fifth aspect, including any embodiment thereof, the effector molecule is a gene editing nuclease such as Cas9.

In an embodiment of the first, second, third, fourth and fifth aspect, including any embodiment thereof, the effector molecule is coding for a therapeutic antibody or a decoy.

In an embodiment of the first, second, third, fourth and fifth aspect, including any embodiment thereof, the effector molecule is effective as a tumor vaccine, preferably the effector molecule is an antigen eliciting an immune response against a tumor cell.

In an embodiment of the first, second, third, fourth and fifth aspect, including any embodiment thereof, the effector molecule is a secreted protein

In an embodiment of the first, second, third, fourth and fifth aspect, including any embodiment thereof, the effector molecule is the effector molecule is effective as an autocrine factor.

In an embodiment of the first, second, third, fourth and fifth aspect, including any embodiment thereof, the effector molecule is effective as an autocrine factor.

In an embodiment of the first, second, third, fourth and fifth aspect, including any embodiment thereof, the effector molecule is effective as a paracrine factor.

In an embodiment of the first, second, third, fourth and fifth aspect, including any embodiment thereof, the recombinant nucleic acid construct consists of ribonucleotides.

In an embodiment of the first, second, third, fourth and fifth aspect, including any embodiment thereof, the recombinant nucleic acid construct consists of deoxyribonucleotides.

In an embodiment of the first, second, third, fourth and fifth aspect, including any embodiment thereof, the recombinant nucleic acid construct consists of ribonucleotides and deoxyribonucleotides.

More specifically, the problem underlying the present invention is solved in a sixth aspect which is also a first embodiment of the sixth aspect, by a vector comprising a recombinant nucleic acid construct of the first aspect, including any embodiment thereof, and/or a recombinant nucleic acid construct as described in connection with each and any aspect, including any embodiment thereof, preferably of the second, third, fourth and fifth aspect.

In a second embodiment of the sixth aspect which is also an embodiment of the first embodiment of the sixth aspect, the vector is an expression vector.

In a third embodiment of the sixth aspect which is also an embodiment of the second embodiment of the sixth aspect, the vector allows expression of the nucleic acid construct in a cell, preferably an endothelial cell, more preferably the vector is a plasmid or a virus.

More specifically, the problem underlying the present invention is solved in a seventh aspect which is also a first embodiment of the seventh aspect, by a cell comprising a recombinant nucleic acid construct according to the first, second, third, fourth and fifth aspect, including any embodiment thereof, and/or a vector according to the seventh aspect, including any embodiment thereof.

In a second embodiment of the seventh aspect which is also an embodiment of the first embodiment of the seventh aspect, the cell is an endothelial cell or a mesenchymal stem cell.

In a third embodiment of the seventh aspect which is also an embodiment of the first and second embodiment of the seventh aspect, the cell is a recombinant cell.

In a fourth embodiment of the seventh aspect which is also an embodiment of the first, second and third embodiment of the seventh aspect, the cell is an isolated cell.

More specifically, the problem underlying the present invention is solved in an eighth aspect which is also a first embodiment of the eighth aspect, by a delivery vehicle comprising a recombinant nucleic acid construct according to the first, second, third, fourth and fifth aspect, including any embodiment thereof, wherein the delivery vehicle is a cationic lipid delivery particle.

In a second embodiment of the eighth aspect which is also an embodiment of the first embodiment of the eighth aspect, the particle is a nanoparticle.

In a third embodiment of the eighth aspect which is also an embodiment of the first and second embodiment of the eighth aspect, the average size of the nanoparticle is from about 30nm to about 200 nm, preferably from about 30 nm to about 140 nm and more preferably from about 30 nm to about 60 nm.

More specifically, the problem underlying the present invention is solved in a ninth aspect which is also a first embodiment of the ninth aspect, by a composition comprising a recombinant nucleic acid construct according to the first, second, third, fourth and fifth aspect, including any embodiment thereof, a vector according to the sixth aspect, including any embodiment thereof, a cell according to the seventh aspect, including any embodiment thereof, and/or a delivery vehicle according to the eight aspect, including any embodiment thereof. In a preferred embodiment, the composition is a pharmaceutical composition comprising a pharmaceutically acceptable excipient.

More specifically, the problem underlying the present invention is also solved in a tenth aspect by the use in the manufacture of a medicament of a recombinant nucleic acid construct according to the first, second, third, fourth and fifth aspect, including any embodiment thereof, a vector according to the sixth aspect, including any embodiment thereof, a cell according to the seventh aspect, including any embodiment thereof, and/or a delivery vehicle according to the eight aspect, including any embodiment thereof. In an embodiment thereof, the medicament is for the treatment of a disease, preferably a disease described herein in connection with the second, third, fourth and fifth aspect, including any embodiment thereof.

More specifically, the problem underlying the present invention is also solved in an eleventh aspect by a method for treating a subject, preferably a human subject, wherein the method comprises administering to the subject a therapeutically effective amount of a recombinant nucleic acid construct according to the first, second, third, fourth and fifth aspect, including any embodiment thereof, a vector according to the sixth aspect, including any embodiment thereof, a cell according to the seventh aspect, including any embodiment thereof, and/or a delivery vehicle according to the eight aspect, including any embodiment thereof. In an embodiment thereof, the medicament is for the treatment of a disease, preferably a disease described herein in connection with the second, third, fourth and fifth aspect, including any embodiment thereof.

More specifically, the problem underlying the present invention is also solved in an twelfth aspect by a method for vaccinating a subject, preferably a human subject, wherein the method comprises administering to the subject an amount effective to elicit in a the subject an immune response, preferably a desired immune response, of a recombinant nucleic acid construct according to the first, second, third, fourth and fifth aspect, including any embodiment thereof, a vector according to the sixth aspect, including any embodiment thereof, a cell according to the seventh aspect, including any embodiment thereof, and/or a delivery vehicle according to the eight aspect, including any embodiment thereof. In an embodiment thereof, the medicament is for the treatment of a disease, preferably a disease described herein in connection with the second, third, fourth and fifth aspect, including any embodiment thereof. In a preferred embodiment, the effector molecule is one which is capable of or suitable for eliciting such immune response.

In an embodiment and as preferably used herein, a heterologous construct or recombinant construct is a construct which as such is not existing in a wild type biological system such as a virus, a cell, a tissue, an organ or an organism. More preferably, a heterologous construct or a recombinant construct is one, where at least one element contained in the construct is not combined with a coding region for the effector, wherein said element is selected from the group consisting of a 5' non-translated region, a 3' non-translated region, a cap structure, a signal sequence and a poly-A tail.

In an embodiment and as preferably used herein, a therapeutic method or a method for the treatment and/or prevention of a disease is a therapy.

In an embodiment of each and any aspect of the present invention, the coding region coding for an effector molecule is expressed by an endothelial cell. In preferred embodiment thereof, the endothelial cell expressing the effector molecule is an endothelial cell the function of which is impaired. In an alternative preferred embodiment, the endothelial cell expressing the effector molecule is an endothelial cell the function of which is not impaired; in such case, the effector molecule is exported or secreted by the effector molecule producing endothelial cell and exerts its effect on any other cell, typically any other endothelial cell, whereby such other cell is one the function of which is impaired. In a preferred embodiment, an impaired function of a cell is function of such cell which is to be restored in according with the present invention in its various aspect.

As preferably used herein, a derivative of a sequence which displays the same function as the parent sequence. In a preferred embodiment, the sequence of the derivative and of the parent case have an identity of at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. It is within the present invention that the derivative is a truncated version of the parent sequence. Such truncation may exist at the 5' end, the 3' end or both the 5' end and the 3' end. If reference is made to a parent sequence in terms of identity, such identity preferable refers to a stretch of subsequent nucleotides shared by both the derivative and the parent case with the sequence of the parent case serving as the reference sequence.

In an embodiment and as preferably used herein, a coding region coding for an effector molecule comprises or consists of a nucleic acid sequence coding to said effector molecule.

In an embodiment and as preferably used herein, a 5' non-translated region is a 5' non-translated sequence.

In an embodiment and as preferably used herein, a 3' non-translated region is a 5' non-translated sequence.

In an embodiment and as preferably used herein, a poly-A tail is a nucleotide sequence comprising or consisting of a plurality of As covalently attached to each other, whereby such covalent linkage is preferably a phosphodiester linkage. In an embodiment thereof, the poly-A tail comprises about 20 to about 240 As (i.e. adenosinphosphates) nucleotides, preferably about 60 to 120 As, more preferable about 100 to 140 As and most preferably about 120 As.

In an embodiment and as preferably used herein, the CAP structure is a nucleotide sequence forming a CAP structure which is known to a person skilled in the art. The poly A tail is thought to stabilize natural messengers and synthetic sense RNA. Therefore, in one embodiment a long poly A tail can be added to an mRNA molecule thus rendering the RNA more stable. Poly A tails can be added using a variety of art-recognized techniques. For example, long poly A tails can be added to synthetic or in vitro transcribed RNA using poly A polymerase (Yokoe, et al. Nature Biotechnology. 1996; 14: 1252-1256). A transcription vector can also encode long poly A tails. In addition, poly A tails can be added by transcription directly from PCR products. Poly A may also be ligated to the 3' end of a sense RNA with RNA ligase (see, e.g., Molecular Cloning A Laboratory Manual, 2nd Ed., ed. by Sambrook, Fritsch and Maniatis (Cold Spring Harbor Laboratory Press: 1991 edition)). In one embodiment, the length of the poly A tail is at least about 90, 200, 300, 400 at least 500 nucleotides. In one embodiment, the length of the poly A tail is adjusted to control the stability of a modified sense mRNA molecule of the invention and, thus, the transcription of protein. For example, since the length of the poly A tail can influence the half-life of a sense mRNA molecule, the length of the poly A tail can be adjusted to modify the level of resistance of the mRNA to nucleases and thereby control the time course of protein expression in a cell. In one embodiment, the stabilized nucleic acid molecules are sufficiently resistant to in vivo degradation (e.g., by nucleases), such that they may be delivered to the target cell without a transfer vehicle.

In a preferred embodiment of the recombinant nucleic acid construct, the recombinant nucleic acid construct is an mRNA.

In a preferred embodiment of the recombinant nucleic acid construct, the recombinant nucleic acid construct is a recombinant nucleic acid molecule.

It is within the present invention defined by each and any aspect, including any embodiment thereof that the nucleotides forming the recombinant nucleic acid molecule may be modified. Suitable modifications include alterations in one or more nucleotides of a codon such that the codon encodes the same amino acid but is more stable than the codon found in the wild-type version of the nucleic acid. For example, an inverse relationship between the stability of RNA and a higher number cytidines (C's) and/or uridines (U's) residues has been demonstrated, and RNA devoid of C and U residues have been found to be stable to most RNases (Heidenreich, et al. J Biol Chem 269, 2131-8 (1994)). In some embodiments, the number of C and/or U residues in an mRNA sequence is reduced. In another embodiment, the number of C and/or U residues is reduced by substitution of one codon encoding a particular amino acid for another codon encoding the same or a related amino acid. Contemplated modifications to the mRNA nucleic acids of the present invention also include the incorporation of pseudouridines and 5'-Methylcytidine. The incorporation of pseudouridines into the mRNA nucleic acids of the present invention may enhance stability and translational capacity, as well as diminishing immunogenicity in vivo. (See, e.g., Kariko, K., et al., Molecular Therapy 16 (11): 1833-1840 (2008)). Substitutions and modifications to the recombinant nucleic acid construct of the present invention, particularly at the level of the individual nucleotide of the recombinant nucleic acid construct, may be performed by methods readily known to one of ordinary skill in the art.

The constraints on reducing the number of C and U residues in a sequence will likely be greater within the coding region of an mRNA, compared to an untranslated region, (i.e., it will likely not be possible to eliminate all of the C and U residues present in the message while still retaining the ability of the message to encode the desired amino acid sequence). The degeneracy of the genetic code, however presents an opportunity to allow the number of C and/or U residues that are present in the sequence to be reduced, while maintaining the same coding capacity (i.e., depending on which amino acid is encoded by a codon, several different possibilities for modification of RNA sequences may be possible). For example, the codons for Gly can be altered to GGA or GGG instead of GGU or GGC.

The term modification also includes, for example, the incorporation of non-nucleotide linkages or modified nucleotides into the nucleic acid sequences of the present invention (e.g., modifications to one or both the 3' and 5' ends of an mRNA molecule encoding a functional protein or enzyme). Such modifications include the addition of bases to a nucleic acid sequence (e.g., the inclusion of a poly A tail or a longer poly A tail), the alteration of the 3' UTR or the 5' UTR, complexing the nucleic acid with an agent (e.g., a protein or a complementary nucleic acid molecule), and inclusion of elements which change the structure of a nucleic acid molecule (e.g., which form secondary structures).

The delivery vehicle according to the eighth aspect is preferably based on a composition of one or more suitable lipids (e.g. liposomes) which in combination with the recombinant nucleic acid construct according to the present invention result in overall cationic-lipid-nucleic acid particles (cLNPs). Such cLNPs can be used for functionally transferring a nucleic acid, particularly an mRNA, into endothelial cells of the mammalian vasculature.

Lipid-nucleic acid nanoparticles (LNPs) formed by complexation of nucleic acids such as the recombinant nucleic acid construct of the present invention with cationic lipids in combination with other lipidic components, such as zwitterionic phospholipids, cholesterol and PEGylated lipids, have already been used to block degradation of RNAs in plasma and to facilitate the functional cellular uptake of the RNAs.

However, in a therapeutic context, particularly in view of parenteral therapeutic applications, the overall charge of the lipid-nucleic acid nanoparticles, characterized by the Zeta-potential, is essential in directing the organ distribution of the nanoparticles. Someone skilled in the art will appreciate that overall neutrally charged LNPs are almost exclusively taken up by organs of the reticuloendothelial system, particularly by the liver and spleen. On the other hand, it is also known to those skilled in the art, that overall positively charged lipid nanoparticles are particularly useful to functionally deliver a pharmaceutical payload into cells of the mammalian vasculature.

Those skilled in the art will also appreciate that free nucleic acids, particularly mRNAs, are immune stimulatory if applied intravenously.

Thus, in view of parenteral therapeutic applications a thorough and stable complexation of the nucleic acid inside the lipidic nanoparticle will be essential in order to prevent undesired immunogenic side reactions. Furthermore, it is also known to those skilled in the art that the size of the lipid-nucleic acid nanoparticle is crucial in suppressing the reticuloendothelial clearance of the particles.

It has recently been described that highly potent limit sized siRNA LNPs (-30 nm mean particle size) can be prepared using a microfluidic mixing process based on a staggered herringbone micromixer (SHM) device.

Due to the millisecond mixing of the lipids and the nucleic acid such as the recombinant nucleic acid construct of the invention on a nanoliter scale, the resulting particles are characterized by a very tight packaging of the nucleic acid inside a dense, solid particle core. On the contrary, those skilled in the art will appreciate that macrofluidic mixing processes using T-type connectors, generally result in larger nanoparticles with a more heterogeneous, multilamellar morphology.

In an embodiment, the present invention provides novel positively charged lipid-nucleic acid nanoparticles comprising a nucleic acid such as recombinant nucleic acid construct of the invention for the functional delivery of said recombinant nucleic acid construct of the invention, particularly mRNA, into cells of the mammalian vasculature, particularly into endothelial cells of the lung vasculature.

In an embodiment of the various aspects of the present invention, including any embodiment thereof, a cationic lipid selected from the group comprising β-(L-Arginyl)-L-2,3-diamino propionic acid-N-palmityl-N-oleyl-amide, L-Arginyl-β-alanine-N-palmityl-N-oleyl-amide, DOTAP, DOTMA or DC-Cholesterol is combined with neutral lipids and further shielding lipid components bearing a polyethylene glycol (PEG) modification. The neutral lipid can be either a zwitterionic phospholipid selected from a group comprising Diphytanoyl-PE, DOPE, DLPE, DMPE, POPE, DSPE or an uncharged sterol lipid selected from a group comprising cholesterol and stigmasterol. The PEGylated lipid component is selected from a group comprising methoxyPEG-DSPE, methoxyPEG-DLG, methoxyPEG-DMG, methoxyPEG-DPG, methoxyPEG-DSG, methoxyPEG-c-DMA, methoxyPEG-C8-Ceramide, methoxyPEG-C16-Ceramide. In a particular embodiment the chain length of the PEG-chain corresponds to a molecular weight in the range of 750 Da to 5000 Da, preferably in a range of 1500 Da to 3000 Da.

In another embodiment of the present invention, the molar ratio of the lipids in the lipid mixture is in a range of 20 - 80 mol% cationic lipid, 10 - 70 mol% neutral lipid and 1 - 10 mol% PEGylated lipid, preferably in a range of 35-65 mol% cationic lipid, 35-65 mol% neutral lipid and 1-5 mol% PEGylated lipid.

In a preferred embodiment, the cationic lipid is β-(L-Arginyl)-L-2,3-diamino propionic acid-N-palmityl-N-oleyl-amide or L-Arginyl-β-alanine-N-palmityl-N-oleyl-amide, the neutral lipid is the phospholipid Diphytanoyl-PE and the PEGylated lipid is methoxyPEG2000-DSPE. The molar ratio is 50 mol% cationic lipid, 49 mol% Diphytanoyl-PE and 1 mol% mPEG2000-DSPE.

In yet another embodiment, the mixture of above mentioned lipids is dissolved in a water miscible solvent selected from a group comprising Ethanol, Acetone, 1-Butanol, 2-Butanol, tert.-Butanol, 3-Methyl-1-Butanol, 2-Methyl-1-Propanol, 1-Propanol, 2-Propanol, Dimethylsulfoxide, preferably from a group comprising Ethanol, tert.-Butanol, 1-Butanol.

The dissolved lipid mixture is subsequently rapidly mixed with a dissolution of a nucleic acid such as the recombinant nucleic acid construct of the invention in an aqueous solvent, resulting in the formation of lipid-nucleic acid nanoparticles (LNPs). In a preferred embodiment of the present invention, the mixing is performed using a microfluidic mixing device, particularly using a staggered herringbone-type mixing chamber, e.g. NanoAssemblr™ (Precision NanoSystems Inc.; Vancouver, Canada) device.

In yet another embodiment, the mass ratio of total lipids to nucleic acid nucleic acid such as the recombinant nucleic acid construct of the invention is in a range of 5 to 60, preferably in a range of 15 to 40 and the volumetric mixing ratio of aqueous nucleic acid solution to organic total lipid solution is in the range of 1:1 to 6:1, preferably in the range of 2:1 to 4:1. The mixing flow-rate of the solutions is in the range of 5 ml/min to 25 ml/min, preferably in a range of 10 ml/min to 20 ml/min.

In a certain embodiment of the invention the organic solvent of the resulting mixture is removed after the mixing step by dialysis or by tangential flow filtration using ultrafiltration membranes, whereby the ultrafiltration membranes can be either hollow fiber membranes or flat-screen membranes. The pore size of the ultrafiltration membrane corresponds to a molecular weight cutoff in the range of 1500 Da to 500.000 Da, preferably in a range of 1500 Da to 100.000 Da and even more preferably in a range of 1500 Da to 30.000 Da. Within the tangential flow filtration step it is also possible to concentrate the LNPs to a desired concentration.

The mean particle size of the resulting LNPs can be measured using dynamic light scattering (DLS) technologies and is preferably in the range of 15 nm to 400 nm, more preferably in a range of 25 nm to 200 nm and even more preferable the particles have a size in the range of 25 nm to 100 nm. The overall particle charge of the LNPs is positive and the Zeta-potential of the particles is preferably in a range between +5 mV to +60 mV, more preferably in a range of +25 mV to +60mV.

In certain embodiments, the delivery vehicle of the invention is a liposomal transfer vehicle, e.g. a lipid nanoparticle or a lipidoid nanoparticle. In one embodiment, the transfer vehicle may be selected and/or prepared to optimize delivery of the recombinant nucleic acid construct of the invention to a target cell. For example, if the target cell is an endothelial cell the properties of the transfer vehicle (e.g., size, charge and/or pH) may be optimized to effectively deliver such transfer vehicle to the target cell, reduce immune clearance and/or promote retention in that target cell. Alternatively, if the target cell is in the central nervous system (e.g., mRNA such as the recombinant nucleic acid construct of the invention administered for the treatment of neurodegenerative diseases may specifically target brain or spinal tissue), selection and preparation of the transfer vehicle must consider penetration of, and retention within, the blood brain barrier and/or the use of alternate means of directly delivering such transfer vehicle to such target cell. In one embodiment, the compositions of the present invention may be combined with agents that facilitate the transfer of exogenous mRNA (e.g., agents which disrupt or improve the permeability of the blood brain barrier and thereby enhance the transfer of exogenous mRNA to the target cells).

Liposomes (e.g., liposomal lipid nanoparticles) are known to be particularly for their use as transfer vehicles of diagnostic or therapeutic compounds in vivo (Lasic, Trends Biotechnol, 16: 307-321, 1998; Drummond et al, Pharmacol. Rev., 51 : 691-743, 1999) and are usually characterized as microscopic vesicles having an interior aqua space sequestered from an outer medium by a membrane of one or more bilayers. Bilayer membranes of liposomes are typically formed by amphiphilic molecules, such as lipids of synthetic or natural origin that comprise spatially separated hydrophilic and hydrophobic domains (Lasic, Trends Biotechnol, 16: 307- 321, 1998). Bilayer membranes of the liposomes can also be formed by amphiphilic polymers and surfactants (e.g., polymerosomes, niosomes, etc.). Such liposomes may also be used as the lipid moiety in the delivery vehicle of the present invention comprising a recombinant nucleic acid construct of the present invention.

It is to be acknowledged that any feature related to the recombinant nucleic acid construct as disclosed in connection with a particular aspect of the present invention is equally an embodiment of the recombinant nucleic acid construct of each and any other aspect of the present invention, including any embodiment thereof. More specifically, any embodiment of the recombinant nucleic acid construct disclosed in connection with the first aspect is also an embodiment of the recombinant nucleic acid construct of the second, third and fourth aspect; similarly, any embodiment of the recombinant nucleic acid construct disclosed in connection with the second, third and fourth aspect is also an embodiment of the recombinant nucleic acid construct of the first aspect.

It is generally acknowledged in the art that the UTRs flanking a coding region do not interfere with the coding region and, more specifically, that the UTRs may, in principle exchanged with interfering with the expression of the coding region (see, e.g. WO 2017/100551 A1; Trepotec et al.; Tissue Engineering Part A, Vol 0 Nr ja (Apr. 2018) (https://doi.org/10.1089/ten.TEA.2017.0485)

The present invention is further illustrated by the following Figs. and Examples form which further features, embodiments and advantages of the present invention may be taken.
Fig. 1 is a schematic representation of conventional mRNA molecule structure.
Fig. 2 is a schematic representation of selected examples for mRNA constructs with the coding sequence with an open reading frame being the one of Nano-luciferase.
Fig. 3 is a restriction map of plasmid pcDNA3.1(-) containing construct PAN11.
Fig. 4 is a restriction digest map of plasmid pdDNA3.1(-) of Fig. 3.
Fig. 5 is an 1% agarose gel stained with EtBr showing the non-linearized (uncut, supercoiled, right lanes) and linearization product (left lanes) of pcDNA3.1(-) plasmids containing constructs PAN28 ("28"), PAN13 ("13"), PAN12 ("12"), PAN11 ("11"), PAN10 ("10"), PAN09 ("09"), PAN08 ("08"), PAN07 ("07") and PAN05 ("05") upon BamHI restriction and tail PCR.
Fig. 6 shows 1% agarose gels stained with EtBr showing PCR products of Poly-A tailing PCRs for the addition of 120 nt of Poly-A tail.
Fig. 7A shows the 5' primers used for the 5' UTRs of the indicated gene; the first column indicates in connection with which construct such primer is to used, the second column indicates the origin of the 5' UTR, and the third column indicates the position where the primer hybridizes to the nucleotide sequence.
Fig. 7B shows the 3' primers used for the 3' UTRs of the indicated gene; the first column indicates in connection with which construct such primer is to used, the second column indicates the origin of the 3' UTR, and the third column indicates the position where the primer hybridizes to the nucleotide sequence.
Fig. 8 is an image of a non-denaturing agarose gel after EtBr staining showing the *in vitro* mRNA transcripts of various construct.

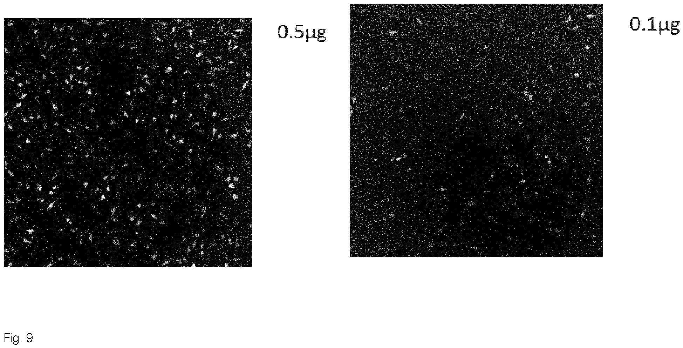

Fig. 9 are photographs of fluorescence microscopy 24 h post transfection of HeLa cells transfected with 0.5 µg (left) and 0.1 µg (right) of a commercially, enhanced mRNA version of the producing green fluorescent protein (TriLINK Biotechnology).
Fig. 10 is a bar diagram showing expression of luciferase, indicated as RLU (relative light units) of luciferase in medium, by the indicated recombinant nucleic acid constructs in HPMEC where luciferase is used as the effector molecule after 2 hours (left column), 6 hours (middle column) and 24 hours (right column) post transfection.
Fig. 11 is a bar diagram showing expression of luciferase, indicated as RLU (relative light units) of luciferase in whole cell lysates of HPMEC, by the indicated recombinant nucleic acid constructs in HPMEC, where luciferase is used as the effector molecule after, 6 hours (left column) and 24 hours (right column) post transfection.
Fig. 12 is a bar diagram showing expression of luciferase, indicated as RLU (relative light units) of luciferase in medium, by the indicated recombinant nucleic acid constructs in HUVEC where luciferase is used as the effector molecule after 2 hours (left column), 6 hours (middle column) and 24 hours (right column) post transfection.
Fig. 13 is a bar diagram showing expression of luciferase, indicated as RLU (relative light units)] of luciferase in whole cell lysates of HUVEC, by the indicated recombinant nucleic acid constructs after lysis of HUVEC, where luciferase is used as the effector molecule after 2 hours (left column), 6 hours (middle column) and 24 hours (right column) post transfection.
Fig. 14 is a bar diagram showing expression of luciferase, indicated as RLU [(relative light units) of luciferase in medium, by the indicated recombinant nucleic acid constructs in HeLa cells where luciferase is used as the effector molecule after 2 hours (left column), 6 hours (middle column) and 24 hours (right column) post transfection.

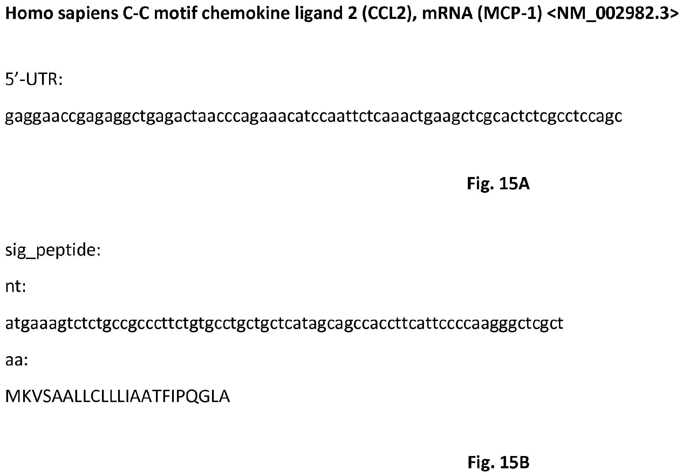

Fig. 15A shows the 5' UTR nucleotide sequence of the mRNA of human MCP-1 which is also referred to as homo sapiens C-C motif chemokine ligand 2 (CCL2) (GeneBank entry NM_002982.3).
Fig. 15B shows both the nucleotide sequence and the amino acid sequence of the signal sequence of human MCP-1.

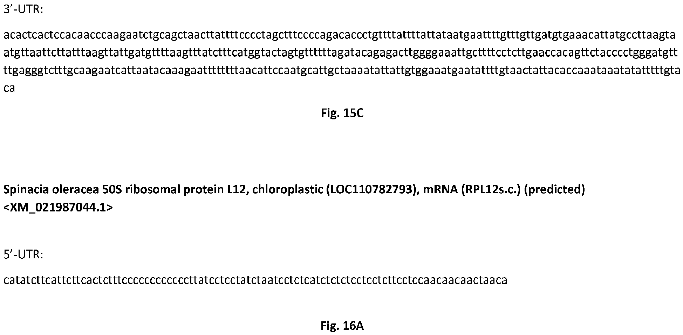

Fig. 15C shows the 3' UTR nucleotide sequence of the mRNA of human MCP-1.
Fig. 16A shows the 5' UTR nucleotide sequence of the mRNA of the 50S ribosomal protein L12, chloroplastic (LOCI 10782793), of Spinacia oleracea which is also referred to as RPL12s.c. (GeneBank entry XM_021987044.1).

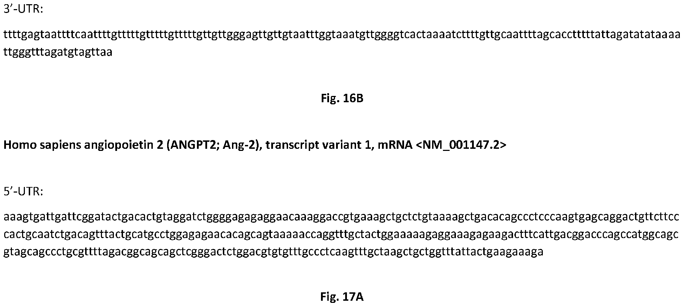

Fig. 16B shows the 3' UTR from the mRNA of the 50S ribosomal protein L12, chloroplastic (LOCI 10782793), of Spinacia oleracea.
Fig. 17A shows the 5' UTR nucleotide sequence of the mRNA of human angiopoietin 2 which is also referred to as ANGPT2 or Ang-2, transcript variant 1(GeneBank entry NM_001147.2).

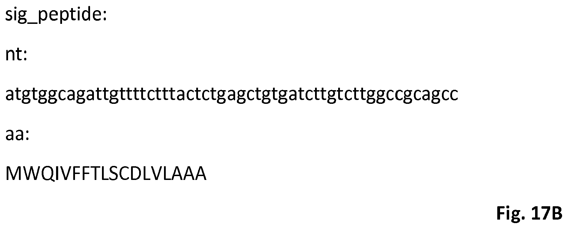

Fig. 17B shows both the nucleotide sequence and the amino acid sequence of the signal sequence of the mRNA of human Ang-2.

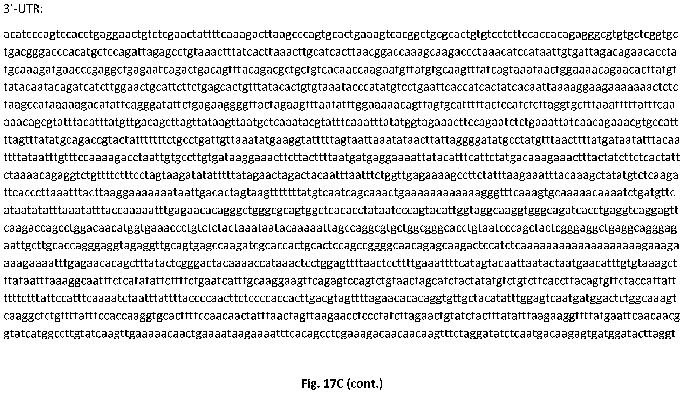

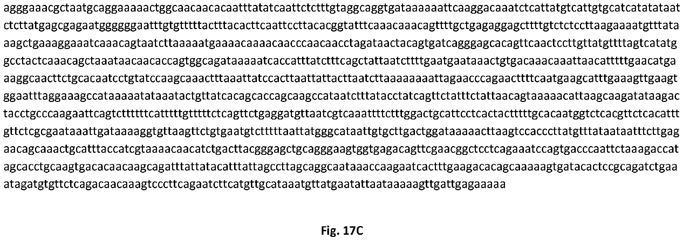

Fig. 17C shows the 3' UTR nucleotide sequence of the mRNA of human Ang-2.

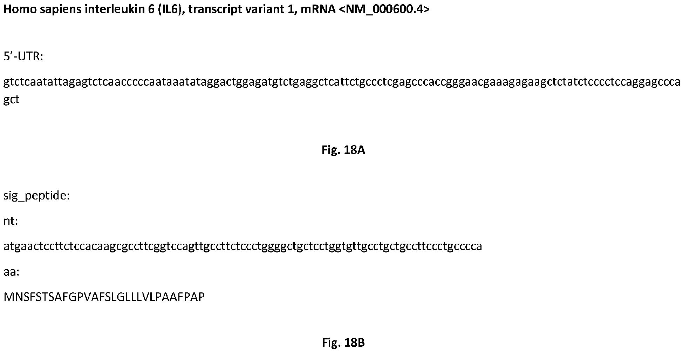

Fig. 18A shows the 5' UTR nucleotide sequence of the mRNA of human interleukin 6 (IL-6), transcript variant 1 (Genebank entry NM_000600.4).
Fig. 18B shows both the nucleotide sequence and the amino acid sequence of the signal sequence of the mRNA of human IL-6.

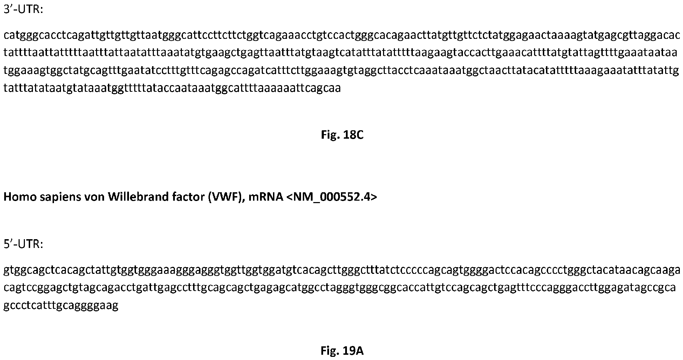

Fig. 18C shows the 3' UTR nucleotide sequence of the mRNA of human IL-6.
Fig. 19A shows the 5' UTR nucleotide sequence of the mRNA of human von Willebrand factor (vWF) (GeneBank entry NM_000552.4).

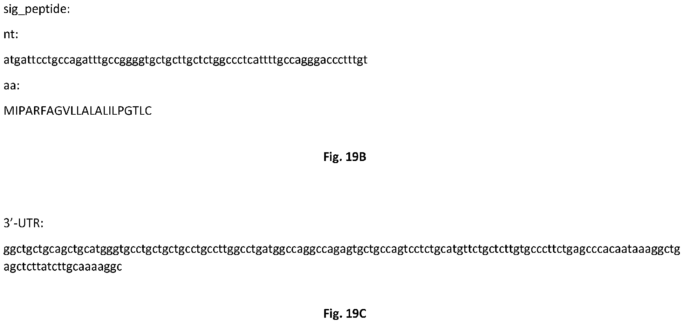

Fig. 19B shows both the nucleotide sequence and the amino acid sequence of the signal sequence of the mRNA of von Willebrand factor (vWF).
Fig. 19C shows the 3' UTR nucleotide sequence of the mRNA of von Willebrand factor (vWF).

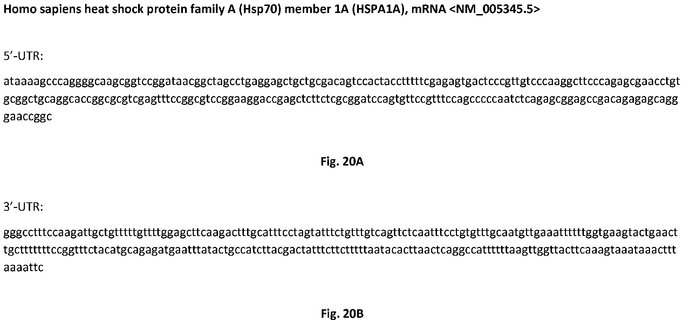

Fig. 20A shows the 5' UTR nucleotide sequence of the mRNA of human heat shock protein family A (Hsp70) member 1A, also referred to as HSPA1A (GeneBank entry NM_005345.5).
Fig. 20B shows the 3' UTR nucleotide sequence of the mRNA of human HSPA1A.
Fig. 21A shows the 5' UTR nucleotide sequence of the mRNA of human heat shock protein family A (Hsp70) member 5, also referred to as HSPA5, (GeneBank entry NM_005347.4).
Fig. 21B shows both the nucleotide sequence and the amino acid sequence of the signal sequence of the mRNA of human HSPA5.

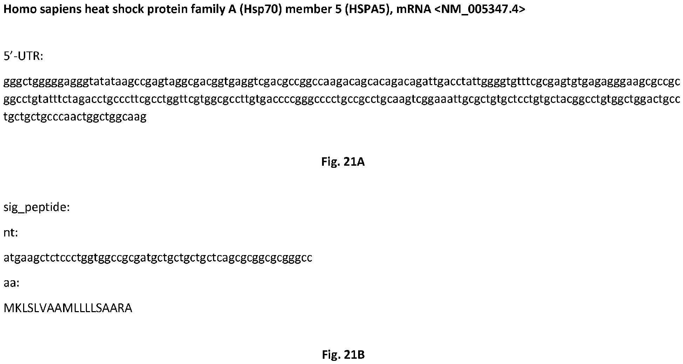

Fig. 21B shows both the nucleotide sequence and the amino acid sequence of the signal sequence of the mRNA of human HSPA5.

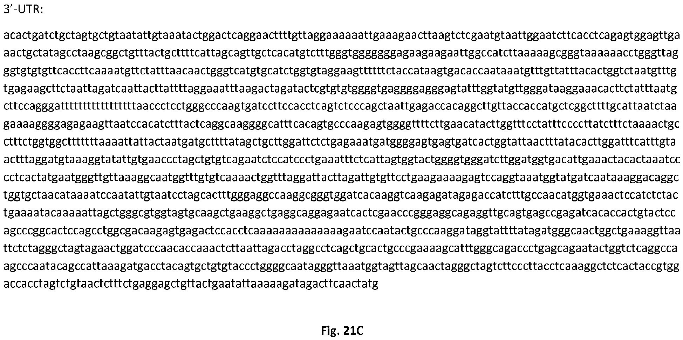

Fig. 21C shows the 3' UTR nucleotide sequence of the mRNA of human HSPA1A.

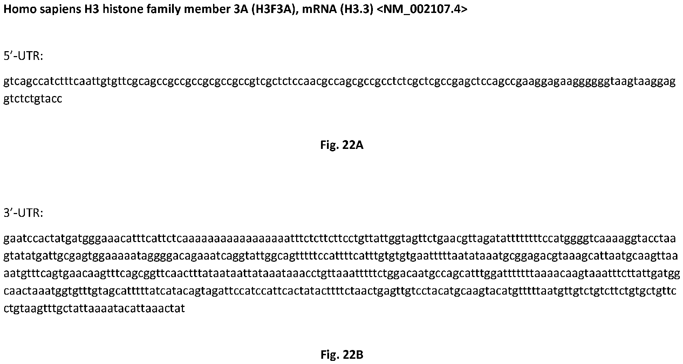

Fig. 22A shows the 5' UTR nucleotide sequence of the mRNA of human H3 histone family member 3A (H3F3A), also referred to as H3.3, (GeneBank entry NM_002107.4).
Fig. 22B shows the 3' UTR nucleotide sequence of the mRNA of human H3.3.

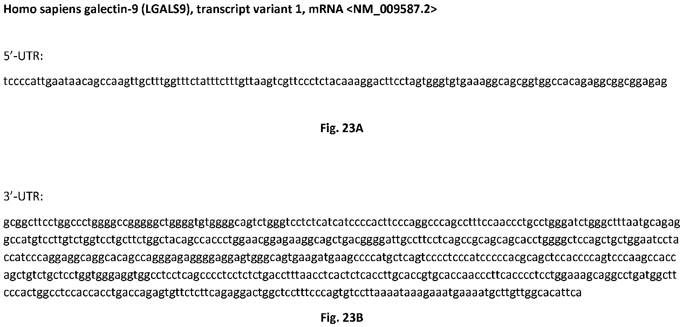

Fig. 23A shows the 5' UTR nucleotide sequence of the mRNA of human galectin-9 (LGALS9), transcript variant 1 (GeneBank entry NM_009587.2).
Fig. 23B shows the 3' UTR nucleotide sequence of the mRNA of human galectin-9.

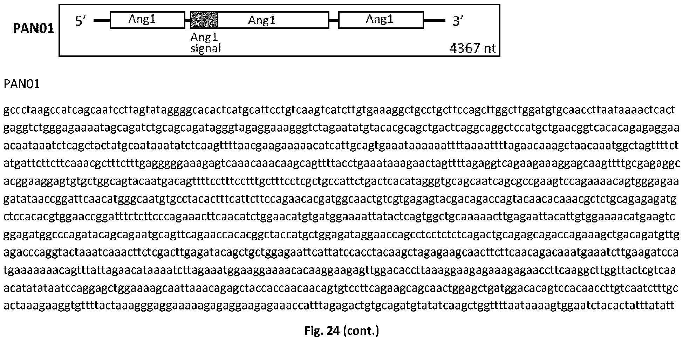

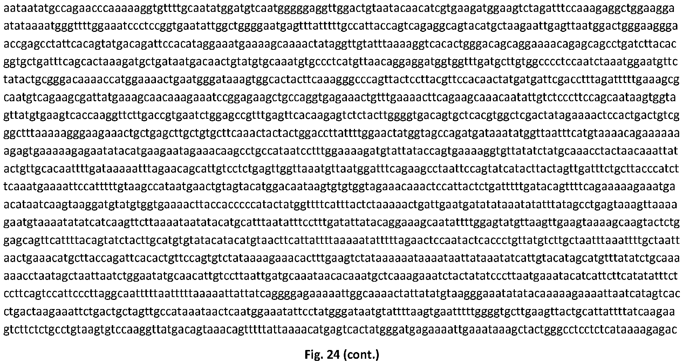

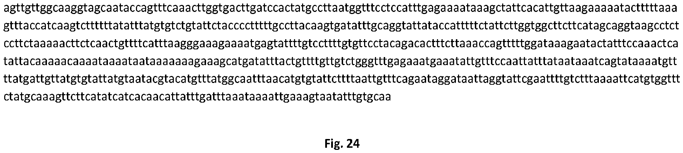

Fig. 24 shows the basic structure and nucleotide sequence of recombinant nucleic acid construct PAN01.

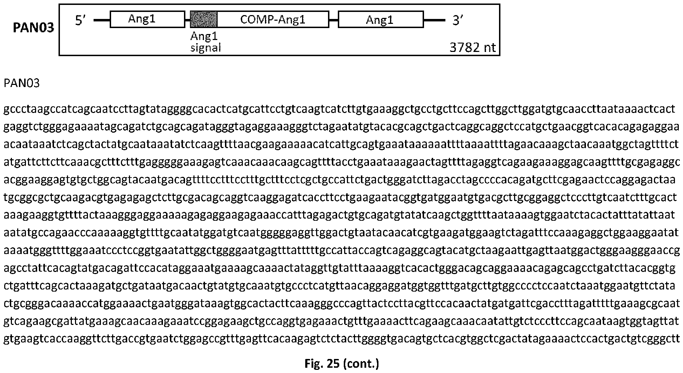

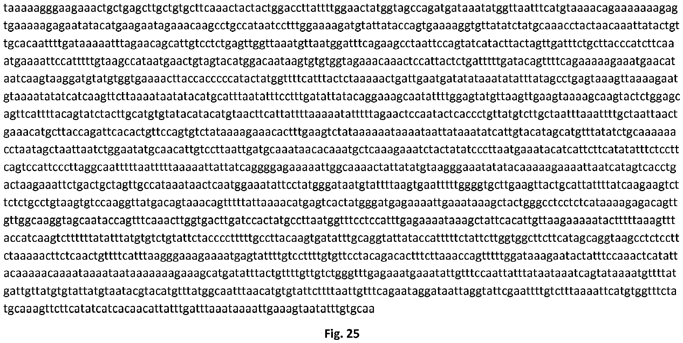

Fig. 25 shows the basic structure and nucleotide sequence of recombinant nucleic acid construct PAN03.

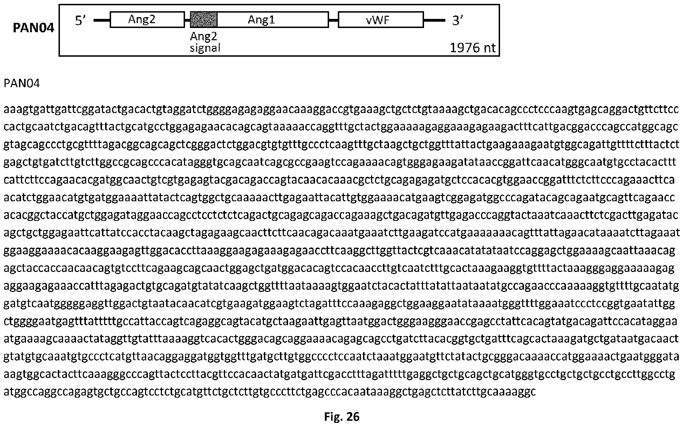

Fig. 26 shows the basic structure and nucleotide sequence of recombinant nucleic acid construct PAN04.

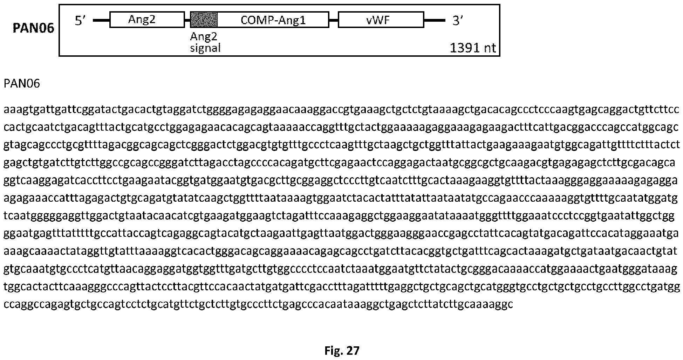

Fig. 27 shows the basic structure and nucleotide sequence of recombinant nucleic acid construct PAN06.

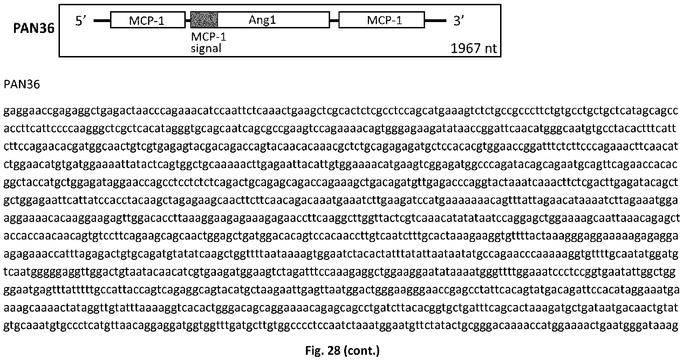

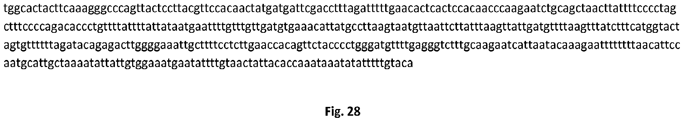

Fig. 28 shows the basic structure and nucleotide sequence of recombinant nucleic acid construct PAN36.

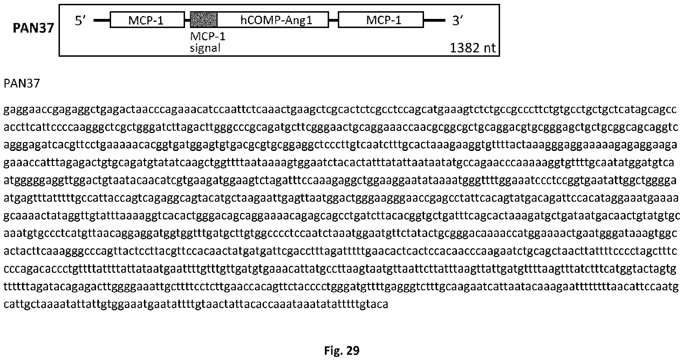

Fig. 29 shows the basic structure and nucleotide sequence of recombinant nucleic acid construct PAN37.

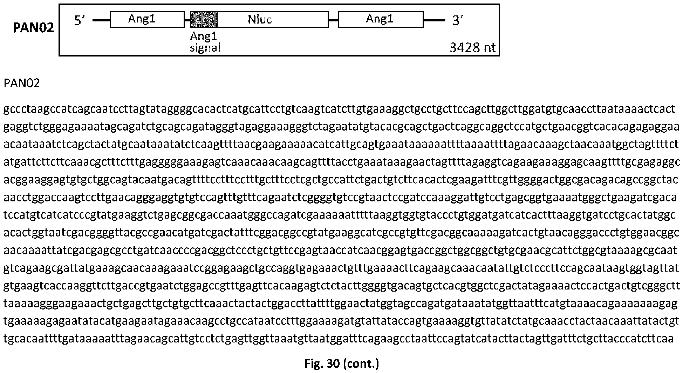

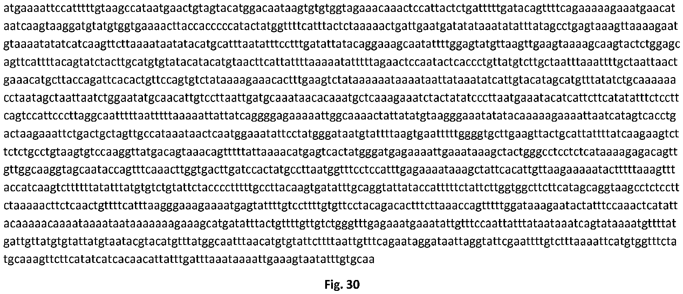

Fig. 30 shows the basic structure and nucleotide sequence of recombinant nucleic acid construct PAN02.

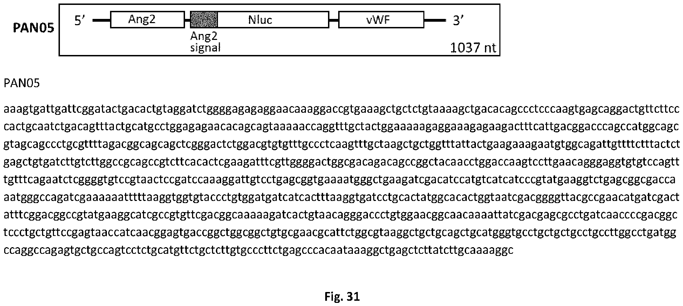

Fig. 31 shows the basic structure and nucleotide sequence of recombinant nucleic acid construct PAN05.

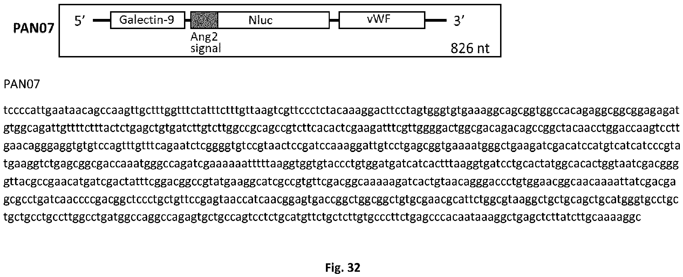

Fig. 32 shows the basic structure and nucleotide sequence of recombinant nucleic acid construct PAN07.

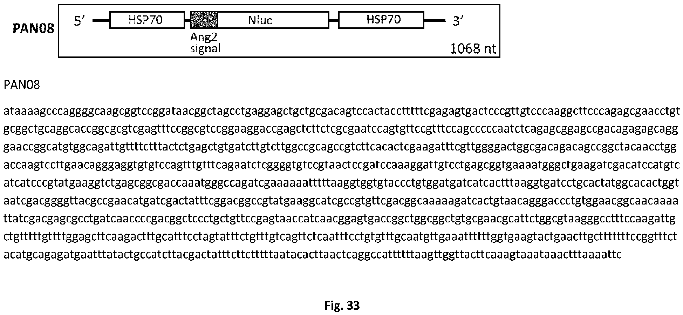

Fig. 33 shows the basic structure and nucleotide sequence of recombinant nucleic acid construct PAN08.

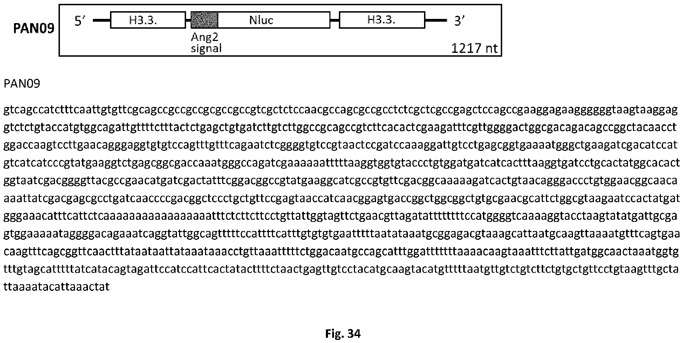

Fig. 34 shows the basic structure and nucleotide sequence of recombinant nucleic acid construct PAN09.

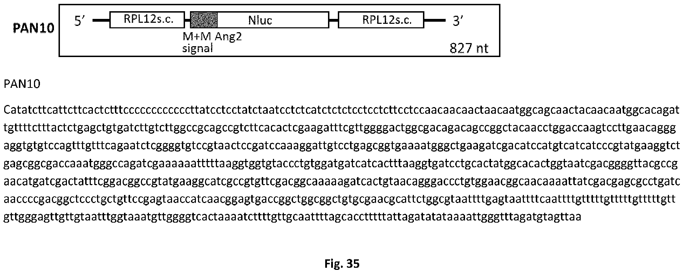

Fig. 35 shows the basic structure and nucleotide sequence of recombinant nucleic acid construct PAN10.

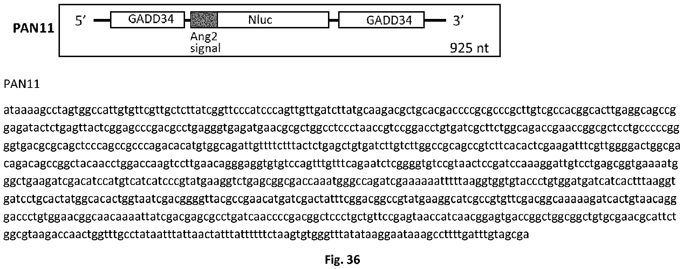

Fig. 36 shows the basic structure and nucleotide sequence of recombinant nucleic acid construct PAN11.

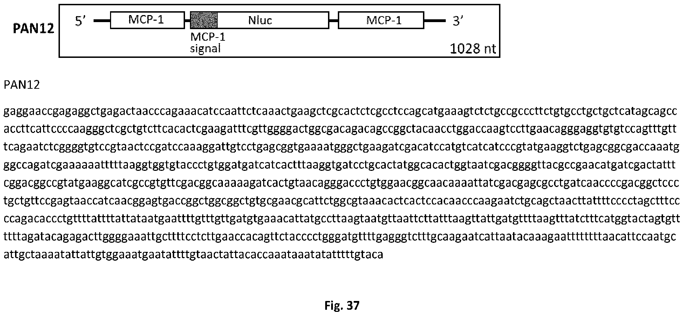

Fig. 37 shows the basic structure and nucleotide sequence of recombinant nucleic acid construct PAN12.

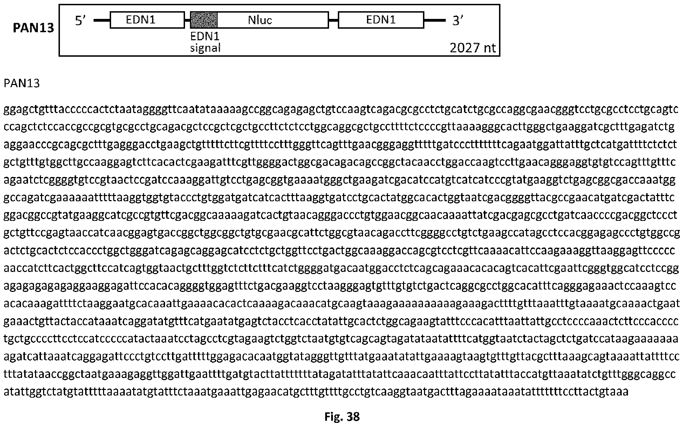

Fig. 38 shows the basic structure and nucleotide sequence of recombinant nucleic acid construct PAN13.

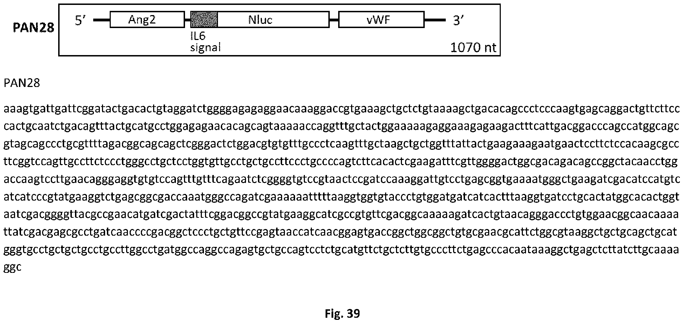

Fig. 39 shows the basic structure and nucleotide sequence of recombinant nucleic acid construct PAN28.

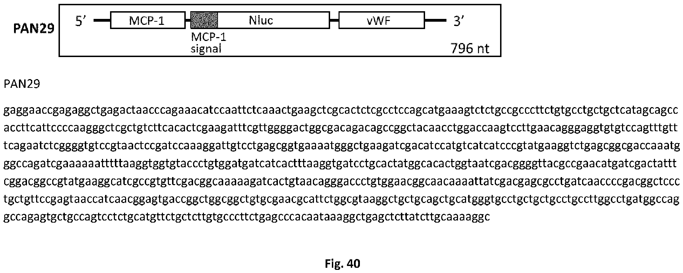

Fig. 40 shows the basic structure and nucleotide sequence of recombinant nucleic acid construct PAN29.

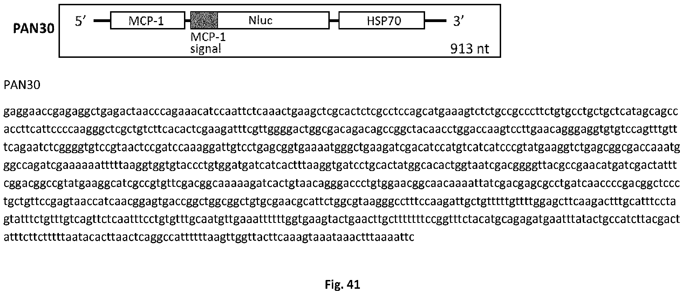

Fig. 41 shows the basic structure and nucleotide sequence of recombinant nucleic acid construct PAN30.

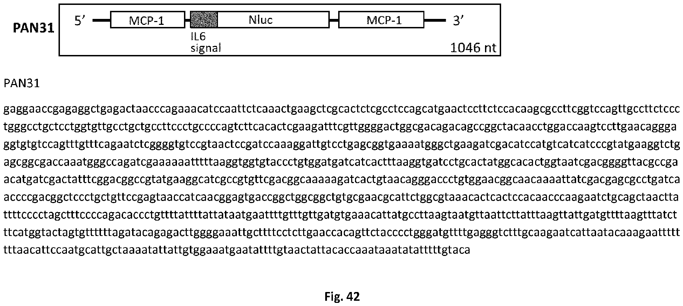

Fig. 42 shows the basic structure and nucleotide sequence of recombinant nucleic acid construct PAN31.

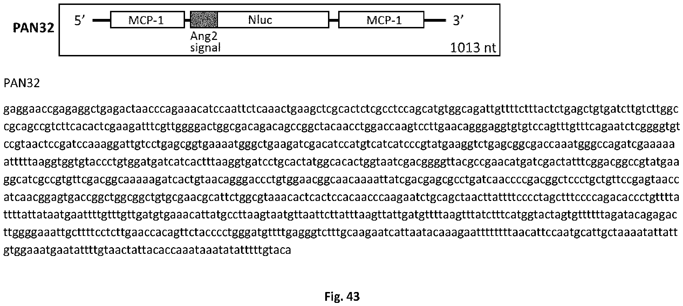

Fig. 43 shows the basic structure and nucleotide sequence of recombinant nucleic acid construct PAN32.

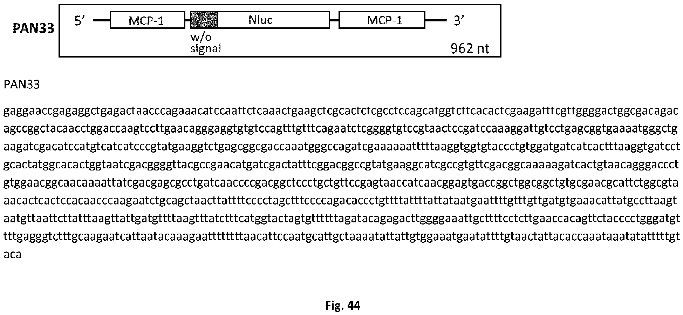

Fig. 44 shows the basic structure and nucleotide sequence of recombinant nucleic acid construct PAN33.

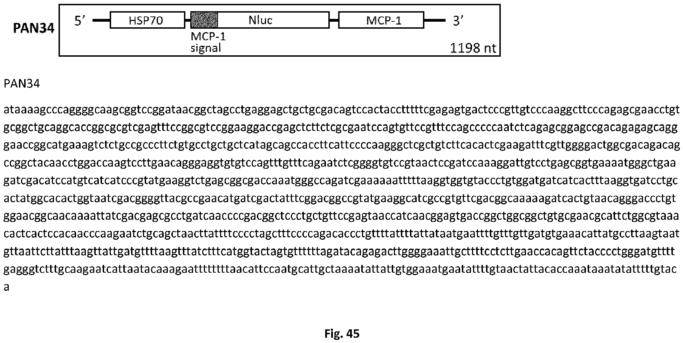

Fig. 45 shows the basic structure and nucleotide sequence of recombinant nucleic acid construct PAN34.

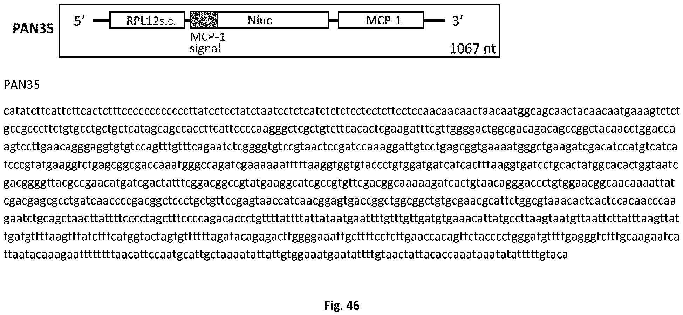

Fig. 46 shows the basic structure and nucleotide sequence of recombinant nucleic acid construct PAN35.

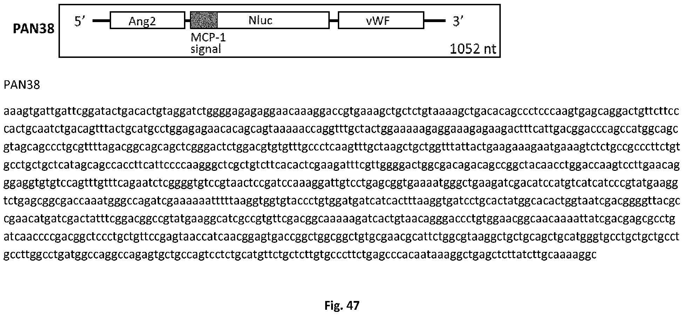

Fig. 47 shows the basic structure and nucleotide sequence of recombinant nucleic acid construct PAN38.

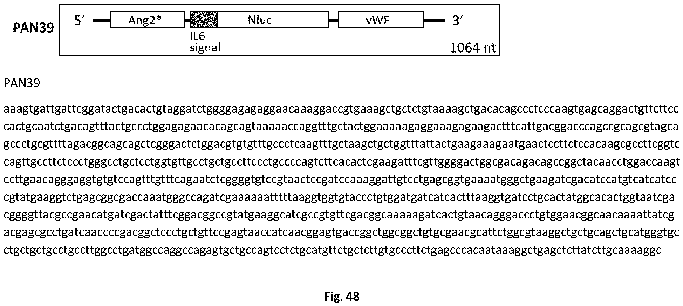

Fig. 48 shows the basic structure and nucleotide sequence of recombinant nucleic acid construct PAN39.

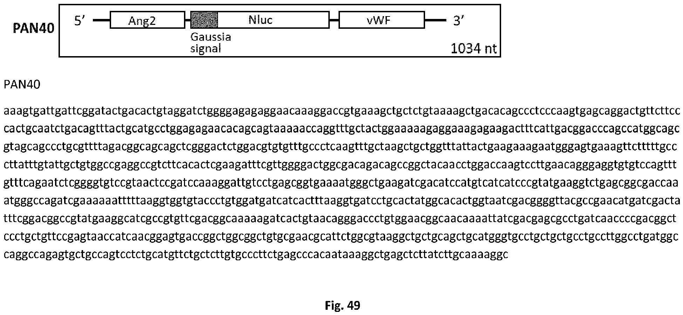

Fig. 49 shows the basic structure and nucleotide sequence of recombinant nucleic acid construct PAN40.

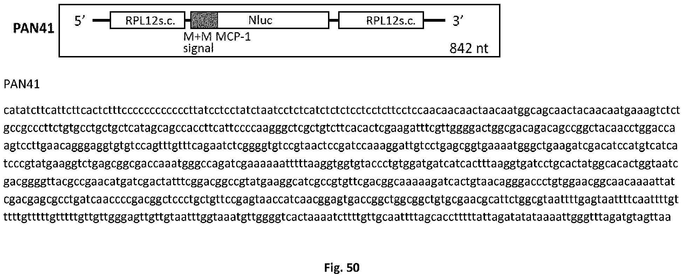

Fig. 50 shows the basic structure and nucleotide sequence of recombinant nucleic acid construct PAN41.

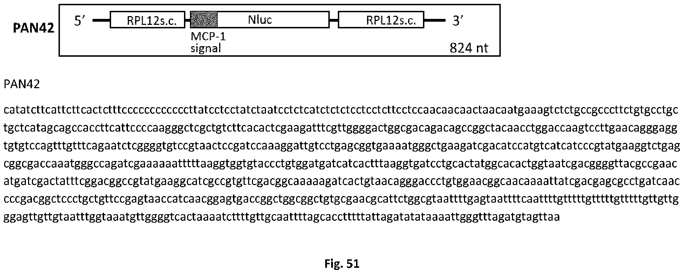

Fig. 51 shows the basic structure and nucleotide sequence of recombinant nucleic acid construct PAN42.

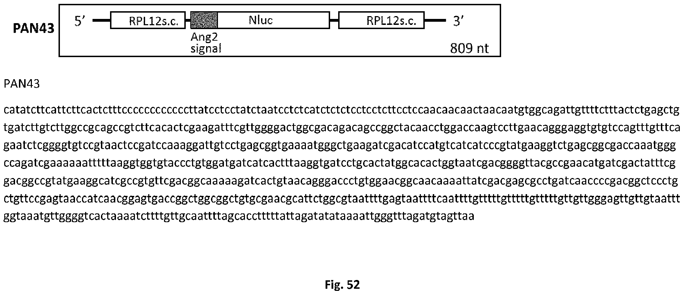

Fig. 52 shows the basic structure and nucleotide sequence of recombinant nucleic acid construct PAN43.

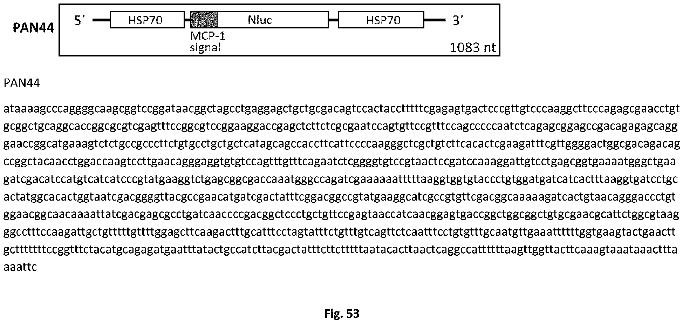

Fig. 53 shows the basic structure and nucleotide sequence of recombinant nucleic acid construct PAN44.

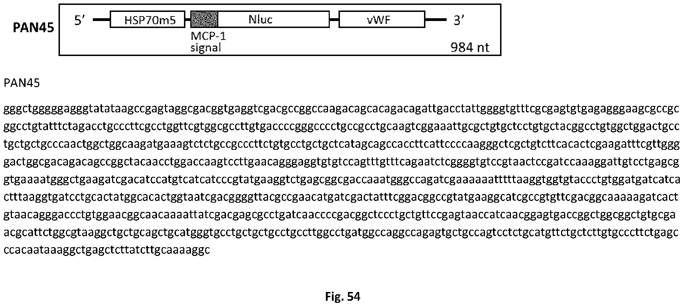

Fig. 54 shows the basic structure and nucleotide sequence of recombinant nucleic acid construct PAN45.

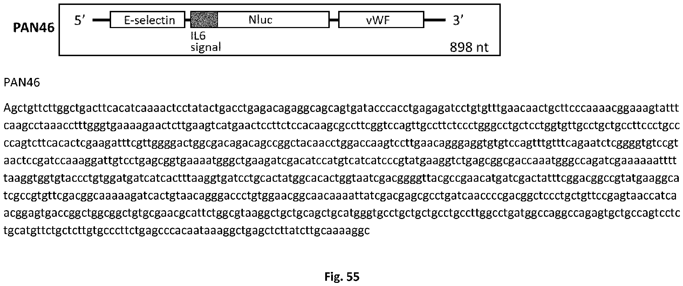

Fig. 55 shows the basic structure and nucleotide sequence of recombinant nucleic acid construct PAN46.

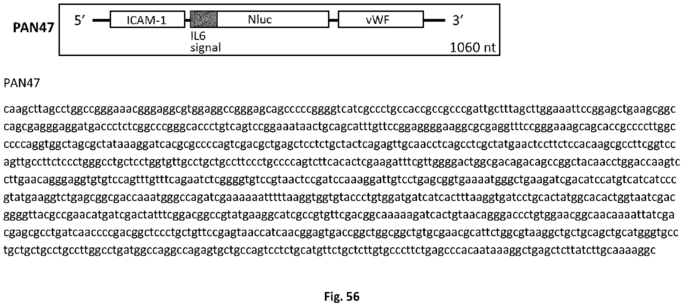

Fig. 56 shows the basic structure and nucleotide sequence of recombinant nucleic acid construct PAN47.

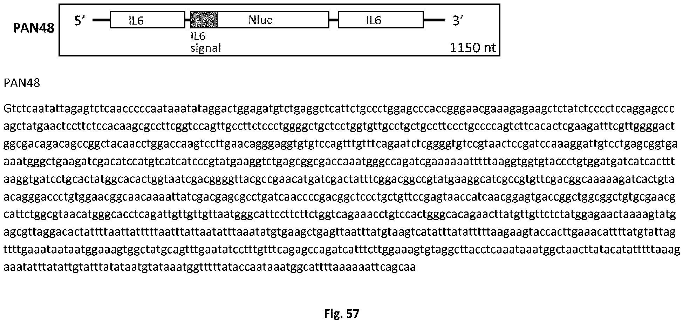

Fig. 57 shows the basic structure and nucleotide sequence of recombinant nucleic acid construct PAN48.

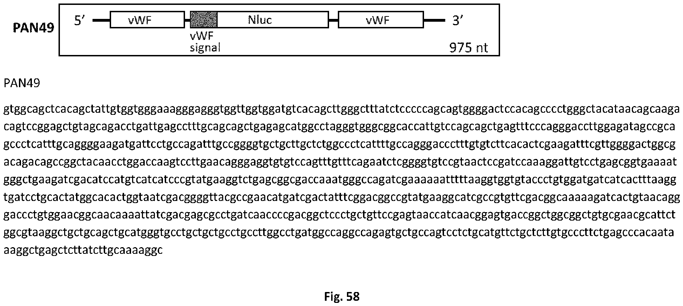

Fig. 58 shows the basic structure and nucleotide sequence of recombinant nucleic acid construct PAN49.

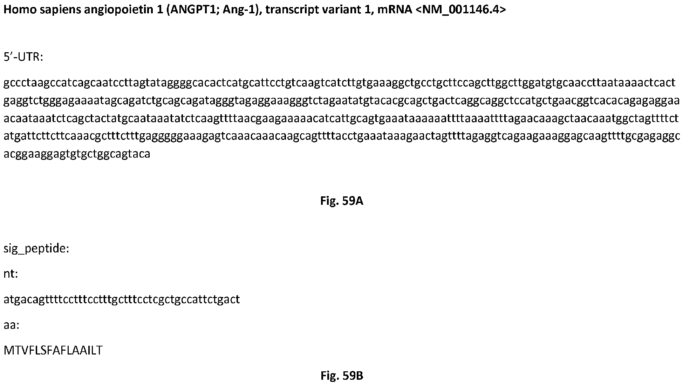

Fig. 59A shows the nucleotide sequence of mRNA of human angiopoietin 1, also referred to as ANGPT1 or Ang-1, transcript variant 1, GeneBank entry NM_001146.4.
Fig. 59B shows both the nucleotide sequence and the amino acid sequence of the mRNA of human Ang-1.

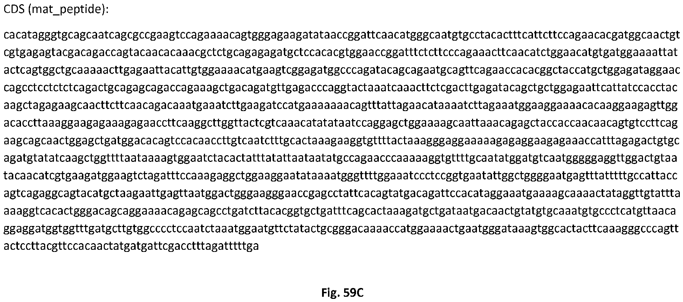

Fig. 59C shows the coding sequence (CDS) of the mature peptide of mRNA of human Ang-1.

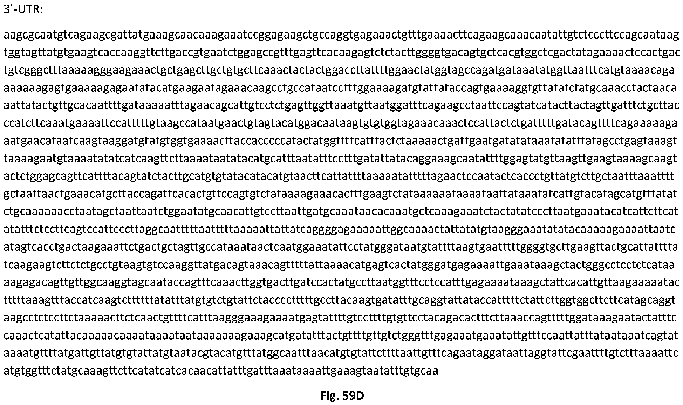

Fig. 59D shows the 3' UTR nucleotide sequence of the mRNA of human Ang-1.

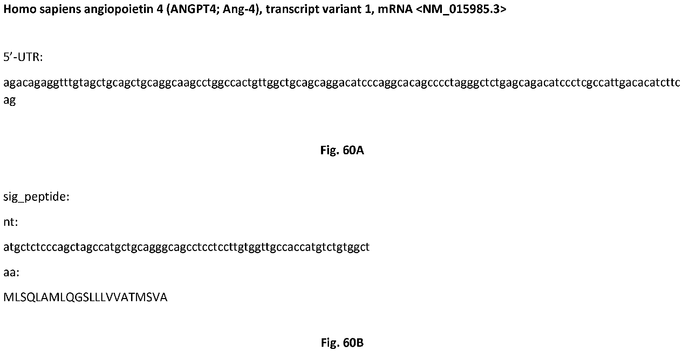

Fig. 60A shows the nucleotide sequence of mRNA of human angiopoietin 4, also referred to as ANGPT4 or Ang-4, transcript variant 1, GeneBank entry NM_015985.3.
Fig. 60B shows both the nucleotide sequence and the amino acid sequence of the mRNA of human Ang-4.

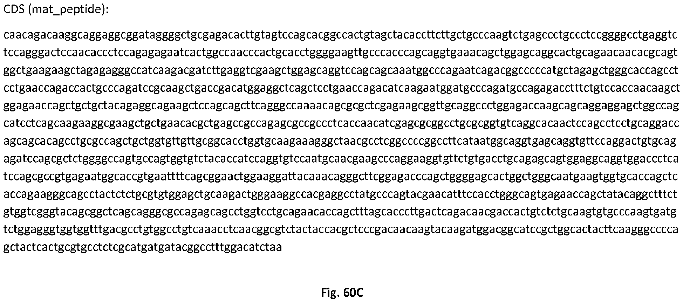

Fig. 60C shows the coding sequence (CDS) of the mature peptide of mRNA of human Ang-4.

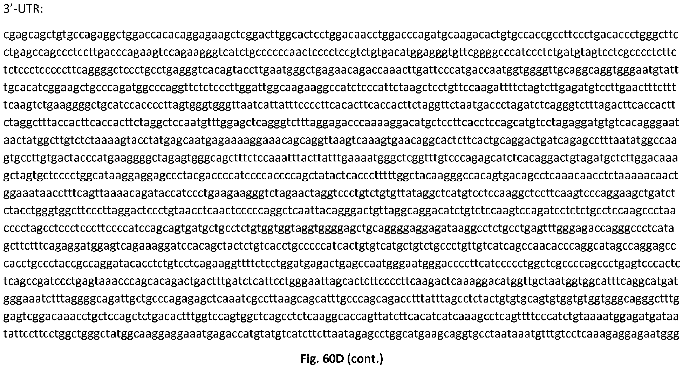

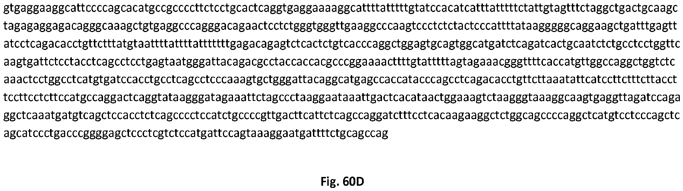

Fig. 60D shows the 3' UTR nucleotide sequence of the mRNA of human Ang-4.

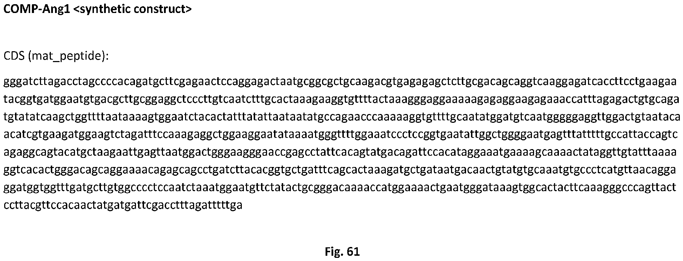

Fig. 61 shows the nucleotide sequence coding for COMP-Angl which is a synthetic construct.

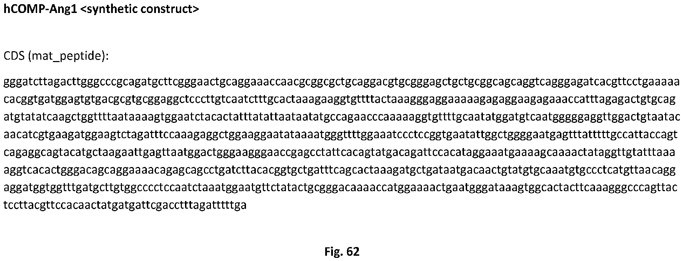

Fig. 62 shows the nucleotide sequence coding for hCOMP-Ang1 which is a synthetic construct.

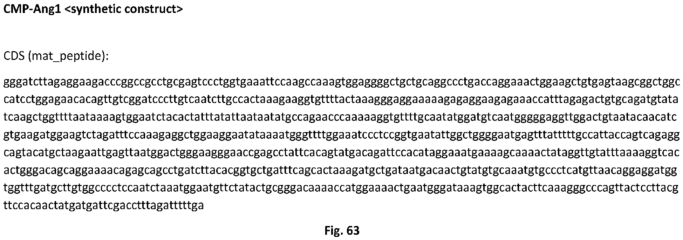

Fig. 63 shows the nucleotide sequence coding for CMP-Ang1 which is a synthetic construct.

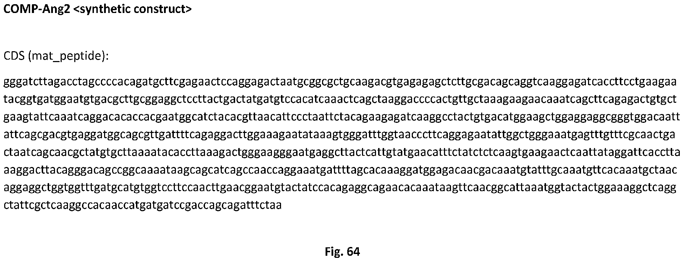

Fig. 64 shows the nucleotide sequence coding for COMP-Ang2 which is a synthetic construct.

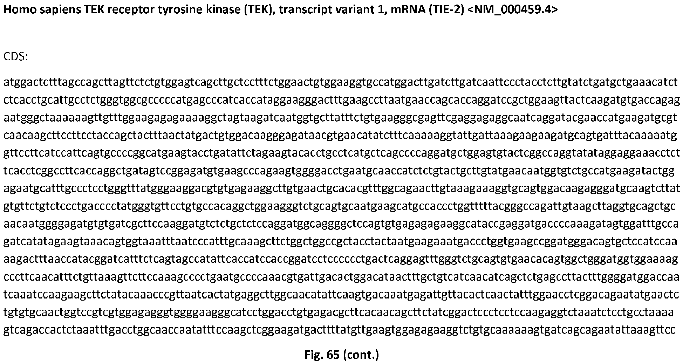

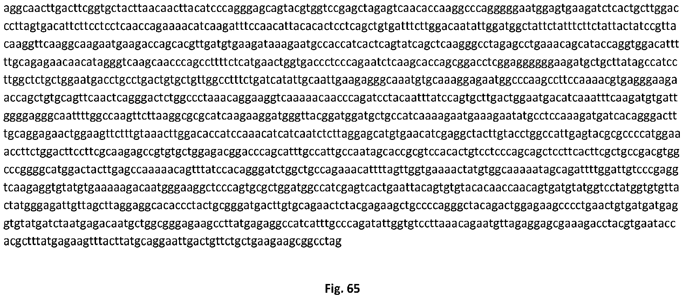

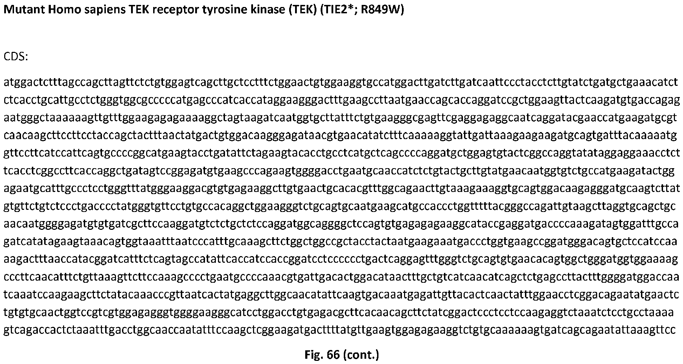

Fig. 65 shows the coding nucleotide sequence of mRNA of human TEK receptor tyrosine kinase (TEK), transcript variant 1, also referred to as TIE-2, GeneBank entry.

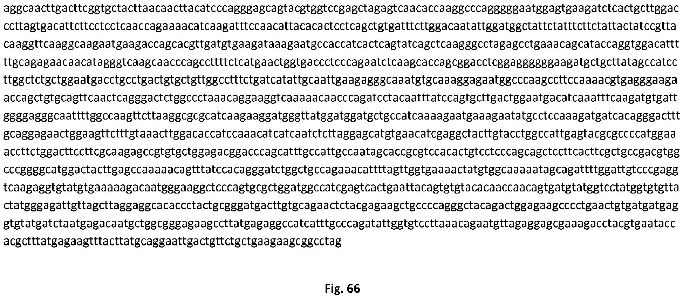

Fig. 66 shows the coding nucleotide sequence of mRNA of human mutant TEK receptor tyrosine kinase (TEK), which is also referred to as TIE* having a R849W mutation.

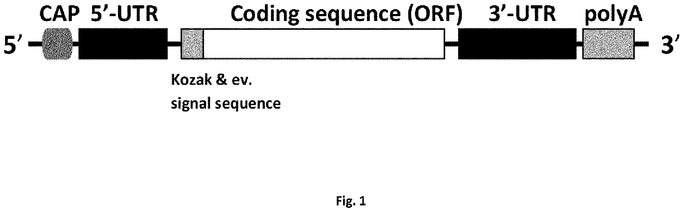

Fig. 1 is a schematic representation of conventional mRNA molecule structure. Eukaryotic including mammalian mRNAs consist of a cap region, a 5' untranslated region (UTR), the coding sequence (CDS) with an open reading frame (ORF) starting with a consensus Kozak sequence for optimal translation initiation and in case of secreted proteins followed by an signal peptide leader sequences, a 3' UTR, and a poly A-tail (>120 nt) at the 3' end.

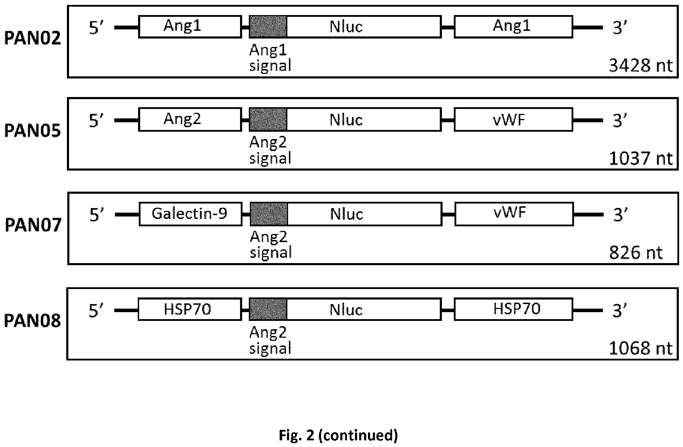

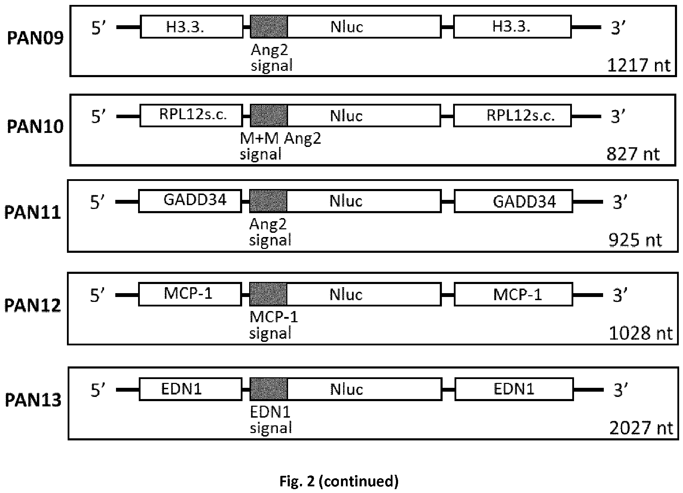

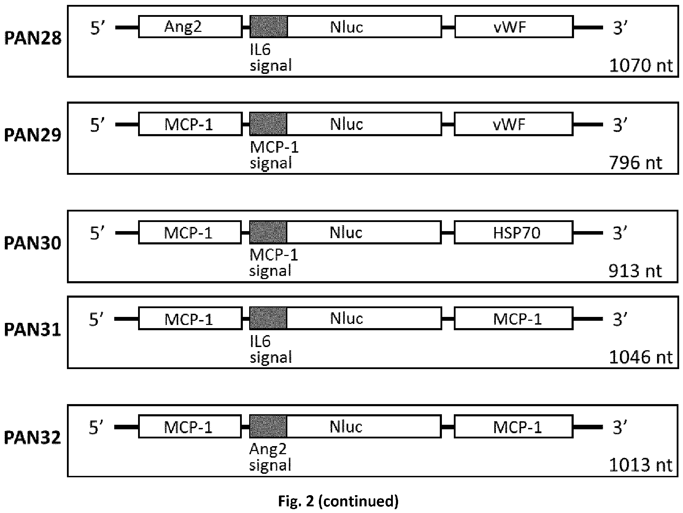

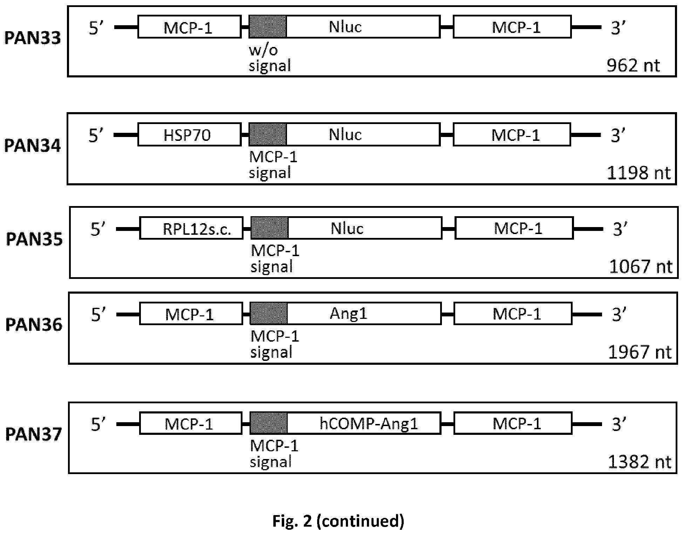

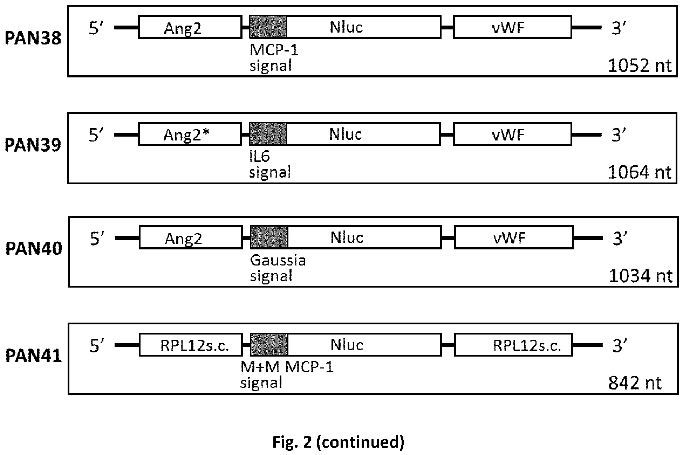

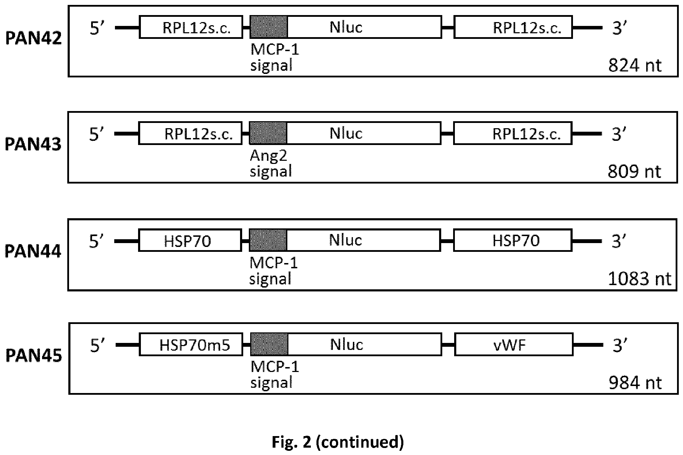

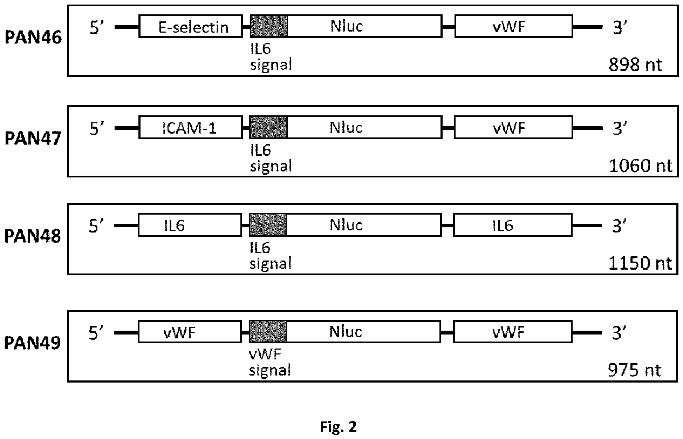

Fig. 2 is a schematic representation of selected examples for mRNA constructs with the coding sequence with an open reading frame being the one of Nano-luciferase. It is within the present invention that for each and any of the indicated mRNA constructs the coding region comprising a sequence with an open reading frame for Nano-luciferase may be replaced by a coding regions comprising a sequence coding for an effector molecule, particularly by a coding region comprising a sequence coding for an effector molecule disclosed herein; in a preferred embodiment thereof, the effector molecule is Ang1 or COMP-Angl. In accordance with the present invention, the recombinant nucleic acid constructs are designed as follows:

Recombinant nucleic acid construct PAN02 comprises as the 5' non-translated region the 5' UTR of Ang1, as the nucleic acid sequence coding for a signal peptide the nucleic acid sequence coding for a signal peptide of Ang1, as the coding region coding for an effector molecule the coding region coding for Nano luciferase, and as the 3' non-translated region the 3' UTR of Ang1.

Recombinant nucleic acid construct PAN05 comprises as the 5' non-translated region the 5' UTR of Ang2, as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of von Willebrand Factor (vWF).

Recombinant nucleic acid construct PAN07 comprises as the 5' non-translated region the 5' UTR of Galectin-9, as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of von Willebrand Factor (vWF).

Recombinant nucleic acid construct PAN08 comprises as the 5' non-translated region the 5' UTR of Hsp70, as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of HSP70.

Recombinant nucleic acid construct PAN09 comprises as the 5' non-translated region the 5' UTR of H3.3, as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of H3.3.

Recombinant nucleic acid construct PAN10 comprises as the 5' non-translated region the 5' UTR of RPL12s.c., as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of RPL12s.c. This construct additionally comprises another start codon preceding the start codon of the coding region for Nano-luciferase in-frame.

Recombinant nucleic acid construct PAN11 comprises as the 5' non-translated region the 5' UTR of GADD34, as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of GADD34.

Recombinant nucleic acid construct PAN12 comprises as the 5' non-translated region the 5' UTR of MCP-1 as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of MCP-1.

Recombinant nucleic acid construct PAN13 comprises as the 5' non-translated region the 5' UTR of EDN1, as the nucleic acid sequence coding for a signal peptide the nucleic acid sequence coding for a signal peptide of EDN1, as the coding region coding for an effector molecule the coding region coding for Nano luciferase, and as the 3' non-translated region the 3' UTR of EDN1.

Recombinant nucleic acid construct PAN28 comprises as the 5' non-translated region the 5' UTR of Ang2, as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of vWF.

Recombinant nucleic acid construct PAN29 comprises as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of von Willebrand Factor (vWF).

Recombinant nucleic acid construct PAN30 comprises as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of HSP70.

Recombinant nucleic acid construct PAN31 comprises as the 5' non-translated region the 5' UTR of MCP-1, as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of MCP-1.

Recombinant nucleic acid construct PAN32 comprises as the 5' non-translated region the 5' UTR of MCP-1, as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of MCP-1.

Recombinant nucleic acid construct PAN33 comprises as the 5' non-translated region the 5' UTR of MCP-1, no nucleic acid sequence coding for a signal peptide, as the coding region coding for an effector molecule the coding region coding for Nano luciferase, and as the 3' non-translated region the 3' UTR of MCP-1.

Recombinant nucleic acid construct PAN34 comprises as the 5' non-translated region the 5' UTR of Hsp70, as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of MCP-1.

Recombinant nucleic acid construct PAN35 comprises as the 5' non-translated region the 5' UTR of RPL12 s.c. (spinach chloroplast), as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of MCP-1.

Recombinant nucleic acid construct PAN36 comprises as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for Ang1, and as the 3' non-translated region the 3' UTR of MCP-1.

Recombinant nucleic acid construct PAN37 comprises as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for hCOMP-Ang1, and as the 3' non-translated region the 3' UTR of MCP-1.

Recombinant nucleic acid construct PAN38 comprises as the 5' non-translated region the 5' UTR of Ang2, as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of von Willebrand Factor (vWF).

Recombinant nucleic acid construct PAN39 comprises as the 5' non-translated region the 5' UTR of Ang2* (Ang2 5'-UTR with deleted upstream ATGs), as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of von Willebrand Factor (vWF).

Recombinant nucleic acid construct PAN40 comprises as the 5' non-translated region the 5' UTR of Ang2, as the nucleic acid sequence coding for a signal peptide the nucleic acid sequence coding for a signal peptide of Gaussia luciferase, as the coding region coding for an effector molecule the coding region coding for Nano luciferase, and as the 3' non-translated region the 3' UTR of von Willebrand Factor (vWF).

Recombinant nucleic acid construct PAN41 comprises as the 5' non-translated region the 5' UTR of RPL12 s.c., as the nucleic acid sequence coding for a signal peptide the nucleic acid sequence coding for a signal peptide of MCP-1 with an additional upstream ATG for translational start, as the coding region coding for an effector molecule the coding region coding for Nano luciferase, and as the 3' non-translated region the 3' UTR of RPL12 s.c.. This construct additionally comprises another start codon preceding the start codon of the coding region for Nano-luciferase in-frame.

Recombinant nucleic acid construct PAN42 comprises as the 5' non-translated region the 5' UTR of RPL12 s.c., as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of RPL12 s.c..

Recombinant nucleic acid construct PAN43 comprises as the 5' non-translated region the 5' UTR of RPL12 s.c., as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of RPL12 s.c..

Recombinant nucleic acid construct PAN44 comprises as the 5' non-translated region the 5' UTR of Hsp70, as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of Hsp70.

Recombinant nucleic acid construct PAN45 comprises as the 5' non-translated region the 5' UTR of Hsp70m5, as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of von Willebrand Factor (vWF).

Recombinant nucleic acid construct PAN46 comprises as the 5' non-translated region the 5' UTR of E-selectin, as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of von Willebrand Factor (vWF).

Recombinant nucleic acid construct PAN47 comprises as the 5' non-translated region the 5' UTR of ICAM1, as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of von Willebrand Factor (vWF).

Recombinant nucleic acid construct PAN48 comprises as the 5' non-translated region the 5' UTR of IL-6, as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of IL-6).

Recombinant nucleic acid construct PAN49 comprises as the 5' non-translated region the 5' UTR of von Willebrand Factor (vWF), as the nucleic acid sequence coding for a signal peptide the nucleic acid sequence coding for a signal peptide of von Willebrand Factor (vWF), as the coding region coding for an effector molecule the coding region coding for Nano luciferase, and as the 3' non-translated region the 3' UTR of von Willebrand Factor (vWF).

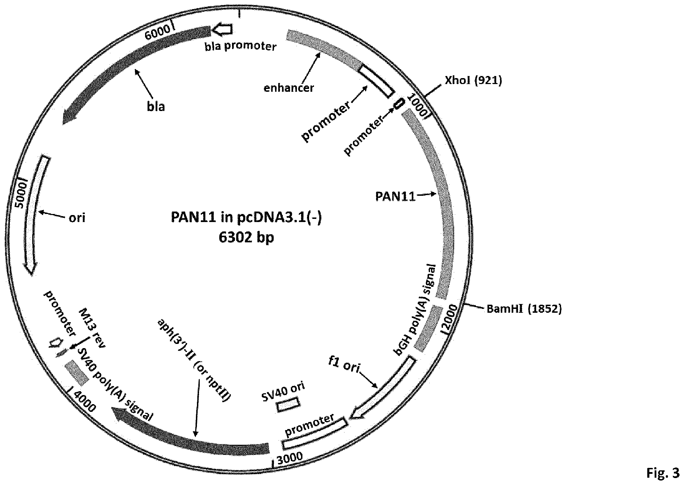

Fig. 3 shows a restriction map of a plasmid pcDNA3.1(-) used as an illustrative example for a plasmid for the expression of an exemplary recombinant nucleic acid construct of the present invention, whereby such exemplary recombinant nucleic acid construct is construct PAN 11; restriction sites XhoI/HindIII are shown.

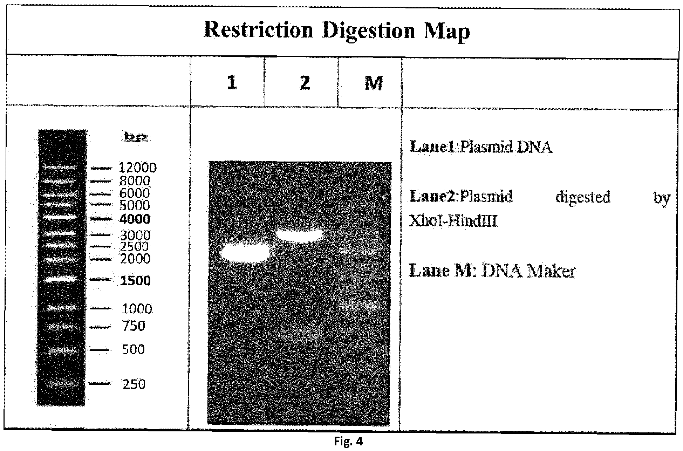

Fig. 4 shows a restriction digestion map of the PAN11 expressing pcDNA3.1- plasmid of Fig. 3; the insert represents PAN11 sequence with engineered 5' and 3' UTR, signal sequence and the coding region coding for Nano-Luciferase.

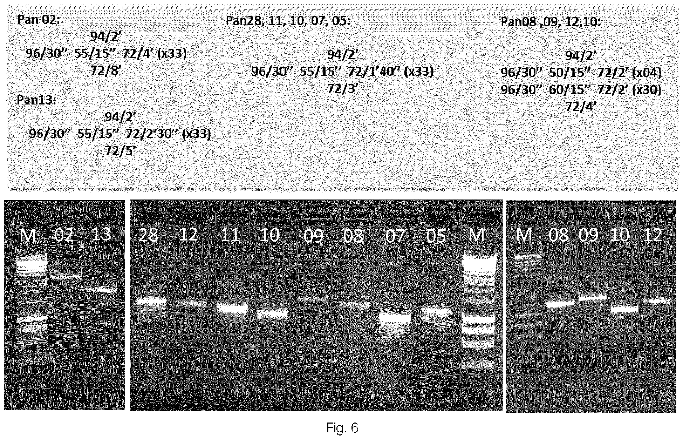

Fig. 6 shows 1% agarose gels stained with EtBr showing PCR products of Poly-A tailing PCRs for the addition of 120 nt of Poly-A tail by 120 nt long poly-T 3' primer flanking the different recombinant nucleic acid constructs. On top are the optimized PCR conditions for the indicated recombinant nucleic acid constructs. For example, for Pan02 the conditions are as follows: denaturation for 2 minutes at 94°C. 33 cycles of 30 seconds at 96°C, 15 seconds at 55°C and 4 minutes at 72°C. Final extension for 8 minutes at 72°C.

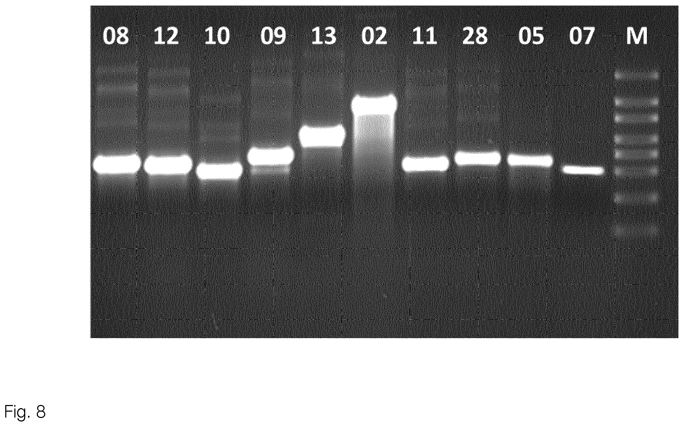

Fig. 8 is an image of a 1% non-denaturing agarose gel stained with EtBr showing the in vitro transcribed mRNAs of constructs PAN28 ("28"), PAN13 ("13"), PAN12 ("12"), PAN11 ("11"), PAN10 ("10"), PAN09 ("09"), PAN08 ("08"), PAN07 ("07") and PAN05 ("05"). M: is a lane with a high range RiboRuler RNA ladder. From this Fig. 8 may be taken that the transcribed mRNAs were intact and, more specifically, were not degraded.

### Example 1: Materials and Methods

### Plasmid Template generation by gene synthesis and cloning

Sequences encoding Nluc and derivatives thereof (e.g. COMP-Ang-1, CMP-Ang-1) proteins with different signal peptides were flanked by different heterologous 5' and 3' UTRs were designed and produced by gene synthesis by BioCat (Heidelberg) and cloned (Xho1-BamHI) into pcDNA3.1- (Thermo Fisher).

### In vitro transcription from PCR products to generate polyadenylated mRNAs

In vitro transcription is the synthesis of RNA transcripts by RNA polymerase from a linear DNA template containing the corresponding promoter sequence (T7, T3, SP6) and the gene to be transcribed. A typical transcription reaction consists of the template DNA, RNA polymerase, ribonucleotide triphosphates, RNase inhibitor and buffer containing Mg2+ ions. Linearized plasmid DNA, PCR products and synthetic DNA oligonucleotides can be used as templates for transcription as long as they have the T7 promoter sequence upstream of the gene to be transcribed. In order to generate DNA templates with a poly-(A) tail a tail-PCR using 5' primers containing T7 RNA polymerase sequences and 3' primers with a 120 nt Poly-T sequence were synthesized and purified (BioSpring, Frankfurt).

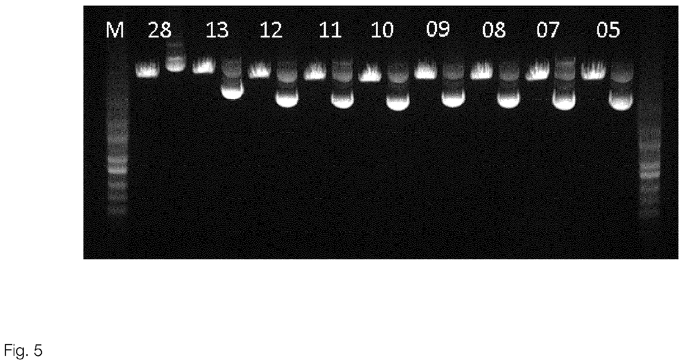

The Linear DNA templates for in vitro transcription were generated by PCR from linearized plasmid. Plasmids were digested with BamHI that cut once 3-terminal in the vector backbone to produce a linearized vector that can be used as the template for the poly-(A) tail PCR. 5µg plasmid are digested for 2h at 37°C with 5U BamHI restriction enzyme in 50µl and 1x HF Buffer. Small Aliquots of digested mix were analyzed by gel electrophoresis to check for complete digestion of the plasmid (see, Fig. 5). The restriction enzyme is heat-inactivated by incubating at 80°C for 20 min and the digested plasmid is purified by a PCR purification column following the manufacturer's protocol (NucleoSpin Gel and PCR clean-up, Macherey-Nagel). The linearized plasmid can be stored at -20OC for several months and can be used for PCR template generation. The purpose of linearization was to eliminate circular templates that could potentially generate run-on transcripts during the IVT reaction.

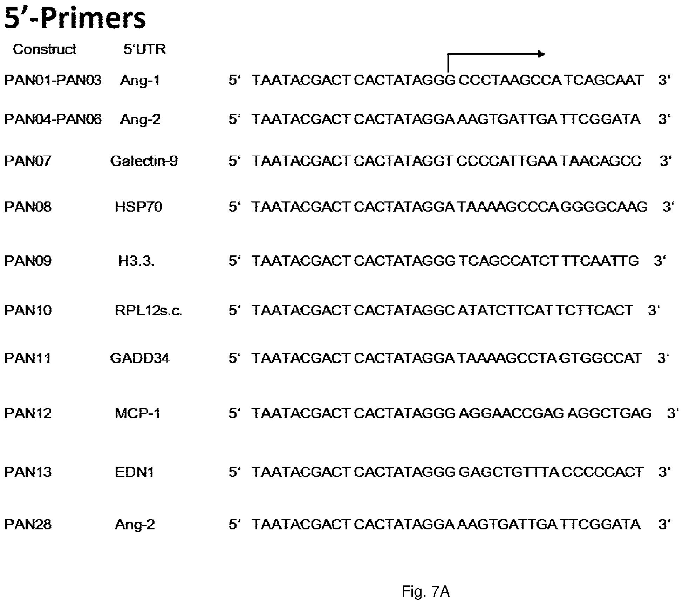

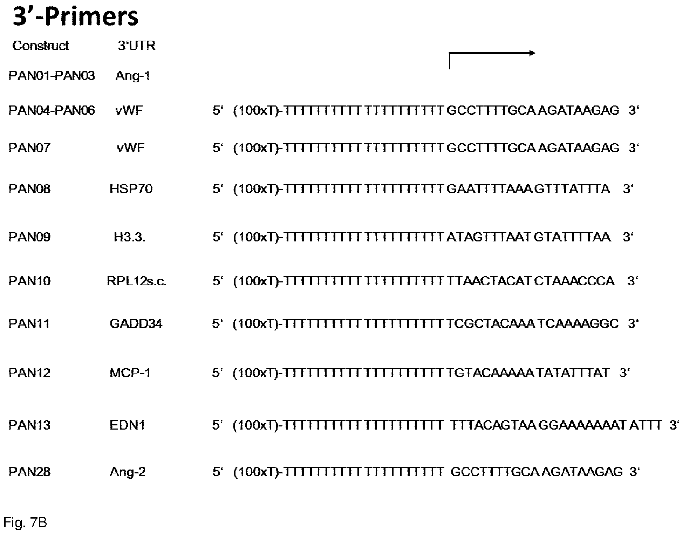

Addition of poly-(A) tail by PCR was performed by using Hot StarHiFidelity polymerase (Qiagen) or Taq DNA Polymerase (Roche) following the manufacturer's protocol. For this purpose, Adapter primers containing T7 promoter sequences and 3' Poly A tail sequences (120nt) were used to amplify linear DNA-templates. Each of the adapter primers has overlap sequences which fused regulatory sequences necessary for translation and transcription to the gene of interest (see Figures 7a and 7b). A typical 50µl PCR reaction contained final concentrations of 200µM (of each) dNTP, 0,5µM for each primer, 50 ng linearized plasmid DNA, 1x PCR reaction buffer (1,5 mM MgCl2) and 1,25 U Taq DNA Polymerase per reaction (0,25µl of /5U/µl). The specific PCR cycle programs or thermal profiles were adjusted according to the Tm of the primer pairs, according to the expected length of the PCR product and according to the used thermal cycler. The quality of the PCR products was checked by analyzing an aliquot by gel electrophoresis and the reactions are purified by commercial PCR purification kits ((NucleoSpin Gel and PCR clean-up, Macherey-Nagel).

### In vitro transcription reaction

Addition of a 5' end cap structure to the RNA is an important process in eukaryotes. It is important for RNA stability, efficient translation, nuclear transport and splicing. The process involved addition of a 7-methylguanosine cap at the 5' triphosphate end of the RNA. RNA capping can be carried out post-transcriptionally using capping enzymes or co-transcriptionally using cap analogs such as ARCA (Jena Bioscience). In the enzymatic method, the mRNA may be capped using the Vaccinia virus capping enzyme. The enzyme adds on a 7-methylguanosine cap at the 5' end of the RNA using GTP and S-adenosyl methionine as donors (cap 0 structure). Both methods yield functionally active capped RNA suitable for transfection or other applications.

In the example described below, the T7 High Yield Transcription Kit (Thermo Scientific) was used to synthesize capped RNA transcripts (PAN 01-XX) using cap analogs co-transriptionally.

The DNA template for the transcription reaction was a linearized plasmid or a PCR product. For the capped RNA the reaction at room temperature was set up in the following order:

| **Component** | **Volume (µl)** | **Final** |
|---|---|---|
| Nuclease free water* | To 20 | |
| 5X Reaction Buffer | 4 | 1X |
| ATP (100 mM) | 2 | 10mM |
| CTP (100 mM) or 5'-Methylcytidine-5'-Triphophate (100 mM) | 2 | 10mM |
| UTP (100 mM) or Pseudouridine-5'-Triphosphate (100 mM) | 2 | 10mM |
| GTP (30 mM) | 2 | 3mM |
| 3'-0-Me-m⁷G(5')ppp(5')G cap analog (ARCA; Anti Reverse Cap Analog) (100 mM) | 2 | 10mM |
| Template DNA* | X | 1 µg |
| T7 RNA Polymerase Mix | 2 | |
| **Total** | **20** | |

1 µg DNA was added and the total reaction volume to 20 µl was obtained by adding nuclease free water. The amount of water to be added varied based on the concentration of the template DNA. The reactions at were well mixed by vortexing and incubated at 37 °C for 2 hours in a dry air incubator. The transcription reactions were treated with DNase I to remove the DNA template before proceeding with purification as follows:
1. 70 µl nuclease free water was added to the transcription reactions followed by 10 µl of DNase I reaction buffer.
2. 2 µl DNase I were added to the reactions.
3. The reaction was incubated at 37 °C for 15 minutes.

### Column Purification of Capped mRNA

1. The capped RNA was purified using the MEGAclearKit as per the manufacturer's instructions (any spin column based RNA purification kit may be used).
2. The RNA was quantified using a NanoDrop Spectrophotometer.
3. As per the manufacturer's instructions, RNA sample quality was assessed using the Agilent RNA 6000 Nano Kit and Agilent 2100 Bioanalyzer or by native 1% agarose gelelectrophoresis and ethidium bromide staining (see, Fig. 8).

### mRNA transfection of HeLa, HPMEC cell and HUVEC cells

HeLa cells, HUVEC, HPMEC cells were grown in DMEM complete medium or Endothelial Cell Growth Medium (PromoCell, Heidelberg Germany) respectively.

RNA transfections were carried out using Lipofectamin MessengerMAX (Invitrogen, Carlsbad, CA; Thermo Fisher Scientific) or TRANSIT -mRNA (Minis Bio, Madison, WI). For mRNA transfection in 24-well plate format 1x105 cells/well were seeded 24h before transfection to reach 70-90 confluence immediate before transfection. For one single well 1.5µl Lipofectamine MessengerMAX Reagent was added to 25µl OptMEM, vortexed and incubated for 10 min at RT. In a second well 500ng - 1µg mRNA were added to 25µl OptiMEM, vortex. After incubation diluted mRNA was added to diluted Lipofectamin, mixed well, incubated for 5 min at RT and added dropwise to cells in the presence of 0,5 ml of DMEM complete medium or Endothelial Cell Growth medium. Cells were incubated at 37°C and transfected cells or serum analyzed at the indicated time points. Alternatively, mRNA-LNPs based on the cationic lipids L-Arginyl-β-alanine-N-palmityl-N-oleyl-amide or β-(L-Arginyl)-L-2,3-diamino propionic acid-N-palmityl-N-oleyl-amide in combination with neutral and PEGylated co-lipids were used for in vitro transfection.

Successful introduction of mRNA into host cells was monitored using various known methods, such as a fluorescent marker, such as Green Fluorescent Protein (GFP), such as reporter enzymes (e.g. luciferase derivatives). Alternatively, transfection of a modified mRNA could also be determined by measuring the protein expression level of the target polypeptide by e.g., Western Blotting or immunocytochemistry or ELISA.

### Nano-Luc Luciferase activity reporter measurement

A time dependent quantitative detection of Nano-Glo-Luciferase expression (Promega) was performed for secreted versions by analyzing samples from the tissue culture supernatant according to the reporter assay technical manual Nano-Glo Luciferase Assay System (Promega). In brief, 5-20µl of serum or lysate were diluted in 100µl H₂O final volume and equal volume of reconstituted Nano-Glo luciferase assay reagent was combined in a 96 well GloMax 96 Microplate. After at least 3 min incubation at RT luminescence were measured in an appropriate luminometer.

### Example 2: Expression of luciferase expressing recombinant nucleic acid constructs in HPMEC, HUVEC and HeLa cells

### mRNAtransfection of HeLa, HUVEC and HPMEC cells

Primary Human Pulmonary Microvascular Endothelial Cells (HPMEC) are most appropriate for studying human lung diseases and are isolated from the lung from a single donor. Since lung tissue contains blood and lymphatic capillaries, HPMEC comprise Blood and Lymphatic Microvascular Endothelial Cells. The cells were routinely analyzed by immunofluorescent staining: they stain positive for CD31 and von Willebrand factor and negative for smooth muscle alpha-actin. HeLa cells, HUVEC, HPMEC cells were grown in DMEM complete medium or Endothelial Cell Growth Medium (PromoCell, Heidelberg Germany, CatNo.: C-22020) respectively. The Endothelial Cell Growth Medium was basal Medium supplemented with Fetal Calf Serum (0.05 ml/ml), Endothelial Cell Growth Supplement (bovine hypothalamic extract; 0.004 ml ml), Heparin 90µg/ml and Hydrocortisone 1µg/ml. The experiments with HUVEC and HPMEC cells were performed on cells with passage numbers below 8.

mRNA transfections were carried out using Lipofectamin MessengerMAX (Invitrogen, Carlsbad, CA; Thermo Fisher Scientific). For mRNA transfection in 24-well plate format 1x10⁵ cells/well were seeded 24h before transfection to reach 70-90 confluence immediate before transfection. For one single well 1.5µl Lipofectamine MessengerMAX Reagent was added to 25µl OpitMEM, vortex and incubated for 10 min at RT. In a second well add 500ng or 1µg mRNA were added to 25µl OptiMEM, vortex. After incubation diluted mRNA was added to diluted Lipofectamin, mixed well, incubated for 5 min at RT and added dropwise to cells in the presence of 0,5 ml of DMEM complete medium for HeLa. For HUVEC and HPMEC the Endothelial Cell Growth medium was replaced and the cells washed with OptiMEM and then added the Lipofectamin MessengerMAxX mRNA complex solution to the well with 0,5 ml OptiMEM prewarmed to 37 °C. Cells were incubated at 37°C for 2h and the OptiMEM medium was replaced with complete Endothelial Cell Growth Medium (PromoCell, Heidelberg Germany) and transfected cells or serum were analyzed at the indicated time points. Alternatively, mRNA-LNPs based on the cationic lipids *L*-Arginyl-β-alanine-*N*-palmityl-*N-*oleyl-amide or β-(*L*-Arginyl)-*L*-2,3-diamino propionic acid-*N*-palmityl-*N*-oleyl-amide in combination with neutral and PEGylated co-lipids can be used for in vitro transfection.

Successful introduction of mRNA into host cells can be monitored using various known methods, such as a fluorescent marker, such as Green Fluorescent Protein (GFP), such as reporter enzymes (e.g. luciferase derivatives) or transfection of a modified mRNA can also be determined by measuring the protein expression level of the target polypeptide by e.g., Western Blotting or immunocytochemistry or ELISA.

### Nano-Luc Luciferase activity reporter measurement

A time dependent quantitative detection of Nano-Glo-Luciferase expression (Promega) was performed for secreted versions by analyzing samples from the tissue culture supernatant according to the reporter assay technical manual Nano-Glo Luciferase Assay System (Promega). In brief, 5-20µl of serum or lysate were diluted in 100µl H₂O final volume and equal volume of reconstituted Nano-Glo luciferase assay reagent was combined in a 96 well GloMax 96 Microplate. After at least 3 min incubation at RT luminescence could be measured in an appropriate luminometer.

### Detection of Ang-1, COM-Ang-1, CMP-Ang-1 protein in cell lysates and supernatant

A time dependent quantitative detection of secreted Ang-1 and secreted COMP-Ang-1 or COMP-Ang-2 expression is performed by analyzing samples from the supernatant tissue culture by ELISA (Human Angiopoietin-1 Quantikine ELISA Kit (DANG10, , R&D systems) or by Western blot according to the technical manual.

500 ng of COMP-Ang-1 (mRNA sequence shown in SEQ ID NO: PAN05) with polyA tail of approximately 120 nucleotides not shown in sequence; 5 'cap,) fully modified with 5-methylcytosine and pseudouridine (COMP-Ang-1, 5mC/pU), fully modified with 5-methylcytosine and Nl-methyl-pseudouridine (COMP-Ang-1,5mC/NlmpU) or unmodified (COMP-Ang-, unmod) is transfected into HUVEC or HPMEC (Human Pulmonary Microvascular Endothelial Cells, PromoCell, Heidelberg) using Lipofectamin MessengerMAX (Invitrogen, Carlsbad, CA; Thermo Fisher Scientific). The supernatant is harvested and run by ELISA 4 hours after transfection to determine the protein expression and cytokine induction.

For immunoblot detection of Ang-1 and derivatives protein lysates were loaded on NuPage SDS-PAGE system (chambers and power supply) with 1.5mm ready-to-use Bis-Tris gels and 4-12% acrylamide gradient with MOPS-buffer as running aid (all Life Technologies, Grand Island, NY). Each lysate sample was prepared to 40ul final volume. This sample contained 25ug protein lysate in variable volume, RIPA buffer to make up volume to 26ul, 4ul of 10x reducing agent and 10µl 4x SDS loading buffer (both from Life Technologies, Grand Island, NY). Samples were heated at 95oC for 5min and loaded on the gel. Standard settings were chosen by the manufacturer, 200V, 120mA and max. 25 W. Run time was 60min, but no longer than running dye reaching the lower end of the gel.

After the run is terminated, the plastic case is cracked and the encased gel transferred to a ready-to-use nitrocellulose membrane kit and power supply (iBLOT; LifeTechnologies, Grand Island, NY). Using default settings, the protein lysate is transferred by high Ampere electricity from the gel to the membrane.

After the transfer, the membranes were incubated in 5% BSA in IX TBS for 15 minutes then in 5% BSA in IX TBS + 0.1 % Tween for another 15 minutes. Primary antibodies (Ang-1 ab183702 Abcam, Cambrige , UK) against human Ang-1 proteins are applied in 3ml of 5% BSA in IX TBS solution at a 1 :500 to 1 :2000 dilution for 3 hours at room temperature and gentle agitation on an orbital shaker. Membranes are washed 3 times with IX TBS/0.1 % Tween, 5 minutes each time with gentle agitation. The secondary antibody (Goat anti-rabbit HRP conjugate; Abeam, Cambridge, MA) is conjugated to horse radish peroxidase and binds to the primary antibody antibodies. The secondary antibody is diluted of 1 : 1000 to 1 :5000 in 5% BSA in IX TBS and incubated for 3 hrs at RT. At the end of incubation time, the membranes are washed 3 times with IX TBS/0.1 % Tween, 5 minutes each time with gentle agitation. The membranes are developed in 5ml Pierce WestPico Chemiluminescent Subtrate (Thermo Fisher, Rockford, IL) as directed. The Western Blot detects protein around the expected size of 57 kd for human Ang-1, and for COMP-Ang-1.

### Results

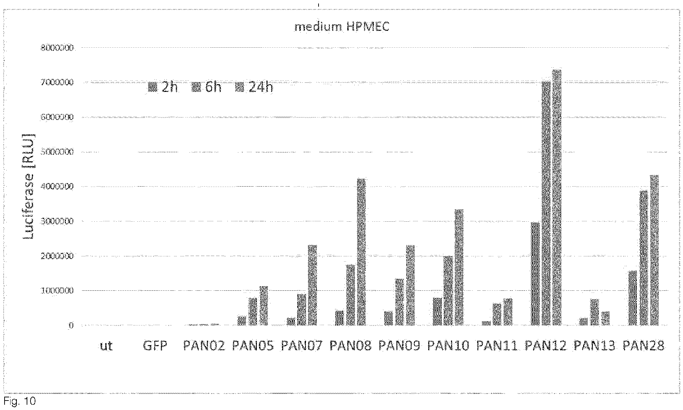

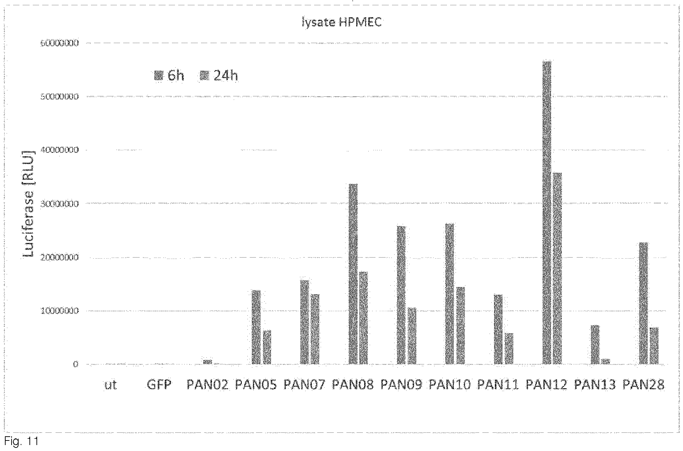

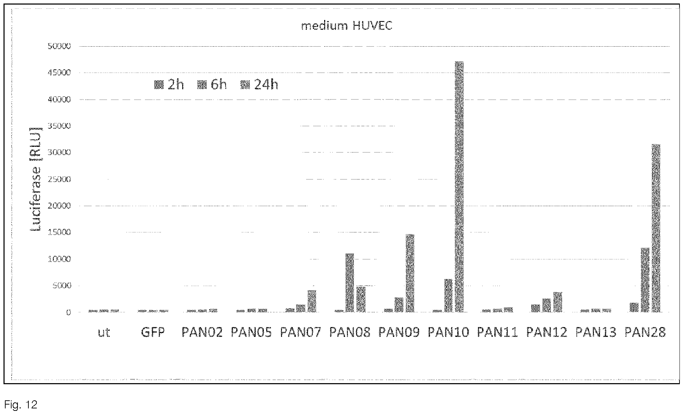

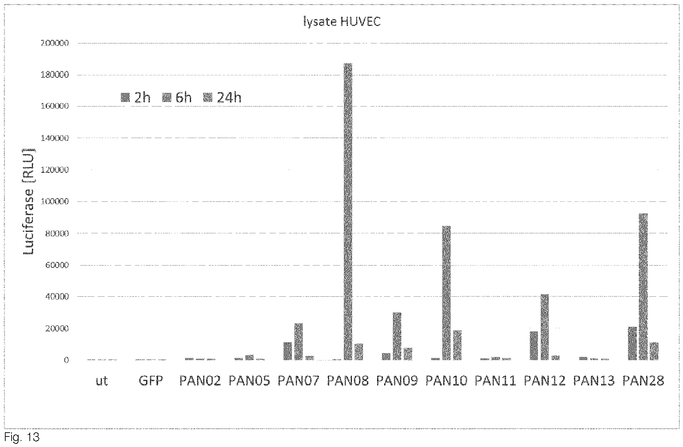

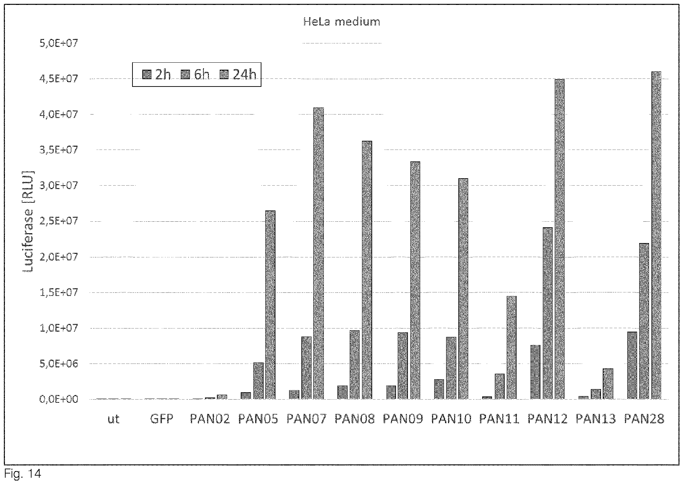

Protein expression data presented in the Figures 10 to 14 underline that combinations of different regulatory nucleotide sequences flanking a coding region can significantly change the protein expression levels. Changes are not only observed in a particular cell type but also across different cell types. For example, PAN02 (wild-type Ang-1 mRNA) shows the lowest Luciferase activity in all cell lines. All other constructs show enhanced protein expressions throughout all cell types. Therefore, the approach of using regulatory sequences from different genes can increase protein expression activity. To this end, replacing the endogenous Ang-1 sequences have shown superior effects on the protein expression of the reporter.

Worth noting, even the same regulatory sequences showed distinct protein expression activity in different cell types. These data further support our approach that the use of specific regulatory sequences can influence the activity of protein expression in a context-specific, i.e. cell-type specific cellular environment.

Furthermore, we have shown that our data are also concerning a different aspect i.e. secretion. For example, the regulatory sequences used in construct PAN12 show cell-type specific (HPMEC) expression in whole cell lysates but also in medium (supernatant). This indicated that these sequences are not only optimal for protein expression (translation) but also secretion. In contrast, construct PAN28 shows expression in all cell types more or less equally.

### Example 3: mRNA Formulation in cationic LNPs for in vivo applications by intravenous administration

For *in vivo* experiments mRNA-LNPs are prepared in a formulation process with *β*-(*L*-Arginyl)-*L*-2,3-diamino propionic acid-*N*-palmityl-*N*-oleyl-amide as cationic lipid. Alternatively, the cationic lipid *L*-Arginyl-β-alanine-*N*-palmityl-*N*-oleyl-amide can be used in an identical procedure to prepare mRNA-LNPs.

mRNA-LNP formulations are prepared using a modified procedure of a method described for siRNA (Chen, S., Tam, Y.Y., Lin, P.J., Sung, M.M., Tam, Y.K., and Cullis, P.R. (2016), Influence of particle size on the in vivo potency of lipid nanoparticle formulations of siRNA. J. Control. Release 235, 236-244. & (ii) patent application US20170121712).

Briefly, lipids are dissolved in ethanol at appropriate molar ratios (e.g. 50:49:1 β-(*L*-arginyl)-*L*-2,3-diamino propionic acid-*N*-palmityl-*N*-oleyl-amide: DPyPE: mPEG2000-DSPE). The lipid mixture is combined with an isotonic Sucrose solution of mRNA at a volume ratio of 2:1 (aqueous:ethanol) using a microfluidic mixer (NanoAssemblr®; Precision Nanosystems, Vancouver, BC) and flow rates of 18ml/min. Similarly, LNP formulations can be obtained using citrate or acetate buffered mRNA solutions (pH 3-4) and a slightly differing mixing ratio of 3:1 (v/v; aqueous:ethanol) and flow rates of 12ml/min.

After the mixing process, the formulations are dialyzed against 10mM HEPES or TRIS buffered isotonic Sucrose solution using 3.5 K MWCO Slide-A-Lyzer Dialysis Cassettes (Thermo Fisher Scientific) for at least 18 hours at 4°C. Instead of Sucrose, other sugars like Trehalose or Glucose can be equally used within the formulation process.

Subsequently, the formulations are tested for particle size (Zetasizer Nano ZS instrument (Malvern Instruments Ltd, Malvern, UK), RNA encapsulation (Quant-iT RiboGreen RNA Assay Kit following manufacturer's (Thermo Fisher Scientific) protocol), and endotoxin and are found to be between 30 to 100 nm in size with a Zeta-potential of >25mV, display greater than 90% mRNA encapsulation and < 1 EU/ml of endotoxin.

mRNA-LNP formulations are stored at -80 °C at a concentration of RNA of ∼ 0.3 µg/µl and an RNA to total lipid ratio of ∼ 0.03-0.05 (wt/wt) until further *in vitro* or *in vivo* use.

The features of the present invention disclosed in the specification, the claims and/or the drawings may both separately and in any combination thereof be material for realizing the invention in various forms thereof.

## Claims

1. A recombinant nucleic acid construct comprising in 5'-> 3' direction
- a 5' UTR,
- a coding region coding for an effector molecule, and
- a 3' UTR,
wherein the 5' UTR is selected from the group comprising a 5' UTR of a gene coding for MCP-1 or a derivative thereof having a nucleotide identity of at least 85 %, a 5' UTR of a gene coding for Ang-2 or a derivative thereof having a nucleotide identity of at least 85 %, a 5' UTR of a gene coding for RPL12s.c. or a derivative thereof having a nucleotide identity of at least 85 %, a 5' UTR of a gene coding for HSP70 or a derivative thereof having a nucleotide identity of at least 85 %, a 5' UTR of a gene coding for H3.3. or a derivative thereof having a nucleotide identity of at least 85 %, a 5' UTR of a gene coding for Galectin-9 or a derivative thereof having a nucleotide identity of at least 85 %, a 5' UTR of a gene coding for GADD34 or a derivative thereof having a nucleotide identity of at least 85 %, a 5' UTR of a gene coding for EDN1 or a derivative thereof having a nucleotide identity of at least 85 %, a 5' UTR of a gene coding for HSP70m5 or a derivative thereof having a nucleotide identity of at least 85 %, a 5' UTR of a gene coding for E-selectin or a derivative thereof having a nucleotide identity of at least 85 % a 5' UTR of a gene coding for ICAM-1 or a derivative thereof having a nucleotide identity of at least 85 %, a 5' UTR of a gene coding for IL-6 or a derivative thereof having a nucleotide identity of at least 85 % and a 5' UTR of a gene coding for vWF or a derivative thereof having a nucleotide identity of at least 85 %;
wherein 3' UTR is selected from the group comprising a 3' UTR of a gene coding for MCP-1 or a derivative thereof having a nucleotide identity of at least 85 %, a 3' UTR of a gene coding for vWF or a derivative thereof having a nucleotide identity of at least 85 %, a 3' UTR of a gene coding for RPL12s.c. or a derivative thereof having a nucleotide identity of at least 85 %, a 3' UTR of a gene coding for HSP70 or a derivative thereof having a nucleotide identity of at least 85 %, a 3' UTR of a gene coding for H3.3. or a derivative thereof having a nucleotide identity of at least 85 %, a 3' UTR of a gene coding for GADD34 or a derivative thereof having a nucleotide identity of at least 85 %, a 3' UTR of a gene coding for EDN1 or a derivative thereof having a nucleotide identity of at least 85 %, and a 3' UTR of a gene coding for IL-6 or a derivative thereof having a nucleotide identity of at least 85 %,
wherein the effector molecule is effective in restoring a cellular function of a cell or is effective in exercising a therapeutic effect in or on a cell, and
wherein the recombinant nucleic acid construct is different from a wild type mRNA coding for the effector molecule.

2. The recombinant nucleic acid construct of claim 1, wherein
a) the 5' UTR is a 5' UTR of a gene coding for HSP70 or a derivative thereof having a nucleotide identity of at least 85 %, and wherein the 3' UTR is selected from the group comprising a 3' UTR of a gene coding for HSP70 or a derivative thereof having a nucleotide identity of at least 85 %, a 3' UTR of a gene coding for RPL12s.c. or a derivative thereof having a nucleotide identity of at least 85 %, a 3' UTR of a gene coding for vWF or a derivative thereof having a nucleotide identity of at least 85 % and a 3' UTR of a gene coding for MCP-1 or a derivative thereof having a nucleotide identity of at least 85 %,
b) the 5' UTR is a 5' UTR of a gene coding for RPL12s.c. or a derivative thereof having a nucleotide identity of at least 85 %, and wherein the 3' UTR is selected from the group comprising a 3' UTR of a gene coding for HSP70 or a derivative thereof having a nucleotide identity of at least 85 %, a 3' UTR of a gene coding for RPL12s.c. or a derivative thereof having a nucleotide identity of at least 85 %, a 3' UTR of a gene coding for vWF or a derivative thereof having a nucleotide identity of at least 85 % and a 3' UTR of a gene coding for MCP-1 or a derivative thereof having a nucleotide identity of at least 85 %,
c) the 5' UTR is a 5' UTR of a gene coding for ANG-2 or a derivative thereof having a nucleotide identity of at least 85 %, and wherein the 3' UTR is selected from the group comprising a 3' UTR of a gene coding for HSP70 or a derivative thereof having a nucleotide identity of at least 85 %, a 3' UTR of a gene coding for RPL12s.c. or a derivative thereof having a nucleotide identity of at least 85 %, a 3' UTR of a gene coding for vWF or a derivative thereof having a nucleotide identity of at least 85 % and a 3' UTR of a gene coding for MCP-1 or a derivative thereof having a nucleotide identity of at least 85 %, or
d) the 5' UTR is a 5' UTR of a gene coding for MCP-1 or a derivative thereof having a nucleotide identity of at least 85 %, and wherein the 3' UTR is selected from the group comprising a 3' UTR of a gene coding for HSP70 or a derivative thereof having a nucleotide identity of at least 85 %, a 3' UTR of a gene coding for RPL12s.c. or a derivative thereof having a nucleotide identity of at least 85 %, a 3' UTR of a gene coding for vWF or a derivative thereof having a nucleotide identity of at least 85 % and a 3' UTR of a gene coding for MCP-1 or a derivative thereof having a nucleotide identity of at least 85 %.

3. The recombinant nucleic acid construct of claim 1, wherein the
a) 3'UTR is a 3' UTR of a gene coding for HSP70 or a derivative thereof having a nucleotide identity of at least 85 % and the 5' UTR is selected from the group comprising a 5' UTR of a gene coding for HSP70 or a derivative thereof having a nucleotide identity of at least 85 % a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %,
b) 3'UTR is a 3' UTR of a gene coding for RPL12s.c. or a derivative thereof having a nucleotide identity of at least 85 % and the 5' UTR is selected from the group comprising a 5' UTR of a gene coding for HSP70 or a derivative thereof having a nucleotide identity of at least 85 % a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %,
c) 3'UTR is a 3' UTR of a gene coding for vWF or a derivative thereof having a nucleotide identity of at least 85 % and the 5' UTR is selected from the group comprising a 5' UTR of a gene coding for HSP70 or a derivative thereof having a nucleotide identity of at least 85 % a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %, or
d) 3'UTR is a 3' UTR of a gene coding for MCP-1 or a derivative thereof having a nucleotide identity of at least 85 % and the 5' UTR is selected from the group comprising a 5' UTR of a gene coding for HSP70 or a derivative thereof having a nucleotide identity of at least 85 %, a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %.

4. The recombinant nucleic acid construct of any one of claims 1 to 3, wherein the construct is one selected from the group comprising
a construct, wherein the 5' UTR is a 5' UTR of a gene coding for MCP-1 or a derivative thereof having a nucleotide identity of at least 85 %, and the 3' UTR is a 3' UTR of a gene coding for MCP-1 or a derivative thereof having a nucleotide identity of at least 85 %,
a construct, wherein the 5' UTR is a 5' UTR of a gene coding for ANG-2 or a derivative thereof having a nucleotide identity of at least 85 %, and the 3' UTR is a 3' UTR of a gene coding for vWF or a derivative thereof having a nucleotide identity of at least 85 %,
a construct, wherein the 5' UTR is a 5' UTR of a gene coding for RPL12s.c. or a derivative thereof having a nucleotide identity of at least 85 %, and the 3' UTR is a 3' UTR of a gene coding for RPL12s.c. or a derivative thereof having a nucleotide identity of at least 85 %,
a construct, wherein the 5' UTR is a 5' UTR of a gene coding for HSP70 or a derivative thereof having a nucleotide identity of at least 85 %, and the 3' UTR is a 3' UTR of a gene coding for HSP70 or a derivative thereof having a nucleotide identity of at least 85 %,
a construct, wherein the 5' UTR is a 5' UTR of a gene coding for H3.3. or a derivative thereof having a nucleotide identity of at least 85 %, and the 3' UTR is a 3' UTR of a gene coding for H3.3. or a derivative thereof having a nucleotide identity of at least 85 %, and
a construct, wherein the 5' UTR is a 5' UTR of a gene coding for Galectin 9 or a derivative thereof having a nucleotide identity of at least 85 %, and the 3' UTR is a 3' UTR of a gene coding for Galectin 9 or a derivative thereof having a nucleotide identity of at least 85 %.

5. The recombinant nucleic acid construct any one of claims 1 to 4, wherein the construct comprises a poly-A tail, preferably at the 3' terminal end of the recombinant nucleic acid construct.

6. The recombinant nucleic acid construct of any one of claims 1 to 5, wherein the construct comprises a CAP structure, preferably at the 5' terminal end of the recombinant nucleic acid construct.

7. The recombinant nucleic acid construct of any one of claims 1 to 5, wherein the construct comprises a IRES (internal ribosomal entry site) sequence, preferably at the 5' terminal end of the recombinant nucleic acid construct.

8. The recombinant nucleic acid construct of any one of claims 1 to 7, wherein the construct comprises nucleic acid sequence coding for a signal peptide, preferably the signal peptide is in-frame with nucleic acid sequence coding for a signal peptide and is arranged between the 5' UTR and the coding region coding for an effector molecule.

9. The recombinant nucleic acid construct of claim 8, wherein the signal peptide allows secretion of the effector molecule.

10. The recombinant nucleic acid construct for use of any one of claims 8 to 9, wherein the nucleotide sequence coding for a signal peptide is selected from the group comprising a nucleotide sequence coding for a signal peptide of MCP-1 or a derivative thereof having a nucleotide identity of at least 85 %, nucleotide sequence coding for a signal peptide of IL-6 or a derivative thereof having a nucleotide identity of at least 85 %, , a nucleotide sequence coding for a signal peptide of Ang-2 or a derivative thereof having a nucleotide identity of at least 85 %, and a nucleotide sequence coding for a signal peptide of Ang-1 or a derivative thereof having a nucleotide identity of at least 85 %.

11. The recombinant nucleic acid construct of any one of claims 1 to 10, wherein the cell a cellular function of which is restored and/or the cell in or on which a therapeutic effect is exercised is an endothelial cell, preferably a vascular endothelial cell, more preferably the vascular endothelial cell is a microvascular endothelial cell.

12. The recombinant nucleic acid construct of any one of claims 1 to 11, wherein the cellular function is one which can be restored by an effector molecule having anti-permeability effect of endothelial cells, an anti-vascular leakage effect, an anti-apoptotic effect of endothelial cells or an anti-inflammatory effect of endothelial cells or an anti-stress response effect, preferably the effect is linked to or associated with the TIE-2 signalling pathway, VEGF-receptor pathway, NOTCH signalling pathway, PI3-kinase pathway, eNOS signalling pathway, sirtuin-dependent metabolic and energy homeostasis pathway, oxidative stress pathway, shear stress response pathway, ET-1 signal transduction pathway, NO-mediated signal transduction pathway, and mechanochemical transduction pathway.

13. A vector comprising a nucleic acid construct of any one of claims 1 to 12.

14. A cell comprising a nucleic acid construct of any one of claims 1 to 12 and/or a vector of claim 13.

15. A delivery vehicle comprising a nucleic acid construct of any one of claims 1 to 12, wherein the delivery vehicle is a cationic lipid delivery particle, preferably the particle is a nanoparticle, more preferably the average size of the nanoparticle is from about 30nm to about 200 nm, preferably from about 30 nm to about 140 nm and more preferably from about 30 nm to about 60 nm.

16. A pharmaceutical composition comprising a nucleic acid construct of any one of claims 1 to 12, a vector of claim 13, a cell of claim 14 and/or a delivery vehicle of claims 15, and a pharmaceutically acceptable diluent.

17. The recombinant nucleic acid construct of any one of claims 1 to 12, for use in a method for the treatment and/or prevention of a disease.

18. The recombinant nucleic acid construct of any one of claims 1 to 12, for use in a method for restoring a cellular function of a cell.
